# Europäisches Patentamt

## European Patent Office

## Office européen des brevets

(19)

(11) Veröffentlichungsnummer: **0 300 008 B1**

(12) # EUROPÄISCHE PATENTSCHRIFT

(45) Veröffentlichungstag der Patentschrift: **23.12.92**

(51) Int. Cl.5: **A61B 5/00**, A61M 5/14, A61F 2/02

(21) Anmeldenummer: **88901205.0**

(22) Anmeldetag: **02.02.88**

(86) Internationale Anmeldenummer:
**PCT/AT88/00005**

(87) Internationale Veröffentlichungsnummer:
**WO 88/05643 (11.08.88 88/18)**

(54) **VORRICHTUNG ZUR BESTIMMUNG INTERESSIERENDER PARAMETER IN LEBENDEN ORGANISMEN.**

(30) Priorität: **02.02.87 AT 201/87**
**09.07.87 AT 1732/87**

(43) Veröffentlichungstag der Anmeldung:
**25.01.89 Patentblatt 89/04**

(45) Bekanntmachung des Hinweises auf die Patenterteilung:
**23.12.92 Patentblatt 92/52**

(84) Benannte Vertragsstaaten:
**AT CH DE FR GB IT LI SE**

(56) Entgegenhaltungen:
**EP-A- 0 070 674      EP-A- 0 134 758**
**WO-A-80/01755      WO-A-81/00888**
**WO-A-81/01794      DE-A- 3 515 624**
**US-A- 4 221 567      US-A- 4 243 030**
**US-A- 4 253 456      US-A- 4 516 580**

**Medical & Biological Engineering and Computing, vol 22, no 5, September 1984 (Staines Middlesex GB) JC Puckup: "Technology and the diabetic patient"pages 385-400**

(73) Patentinhaber: **AVL Medical Instruments AG**
**Stettemerstrasse 28**
**CH-8207 Schaffhausen(CH)**

(72) Erfinder: **SKRABAL, Falko**
**Goethestrasse 13**
**A-8010 Graz(AT)**

(74) Vertreter: **Krause, Walter, Dr. Dipl.-Ing. et al**
**Postfach 200 Singerstrasse 8**
**A-1010 Wien(AT)**

**Beschreibung**

Die Erfindung betrifft eine Vorrichtung zur Bestimmung der Konzentration von zumindest einer in lebenden Organismen vorliegenden Substanz, mit einer Einrichtung zur Zu- und Abfuhr einer Perfusionsflüssigkeit, welche eine in den Organismus einbringbare Hohlnadel aufweist, sowie mit einer Auswerteeinheit.

Für medizinische Zwecke besteht häufig die Notwendigkeit einer oftmaligen oder kontinuierlichen Analyse der Zusammensetzung der Körperflüssigkeiten, um Störungen der Homöostase zu erfassen und beseitigen zu können. Um häufiges Blutabnehmen unnotwendig zu machen, gibt es seit vielen Jahren Versuche, Sensoren im Körper von Patienten zu plazieren um laufend Informationen zu erhalten. Ein Beispiel für die Verwendung von an der Oberfläche eines Ösophagus-Katheters angebrachten Sensoren ist z.B. aus der EP-A 101 595 bekannt.

Sich im Körper befindliche Sensoren überziehen sich nachteiliger Weise innerhalb kurzer Zeit mit Gerinnungsprodukten oder Zellen, sodaß die Messungen ungenau werden. Ein weiterer Nachteil ist dadurch gegeben, daß sich in den Körper eingebrachte Sensoren meist der notwendigen Nachkalibrierung entziehen.

Eine besonders gefragte Anwendung für die kontinuierliche Messung ist die Bestimmung der Glucosekonzentration im Organismus. Wegen der methodischen Schwierigkeiten bei der laufenden Registrierung der Glucose wurde in der US-PS 4 403 984 ein Verfahren beschrieben, die Glucosekonzentration indirekt über die Messung von zwei anderen Parametern, z.B. Osmolarität und Leitfähigkeit zu ermitteln.

Aus den US-PSen 4 221 567, 4 253 456 und 4 516 580 sind Verfahren und Vorrichtungen zur Dialyse bekannt, bei welchen zumindest eine Kanüle oder Katheter in den Blutstrom eingebracht wird. Über Dialysemembranen wird eine komplette Equilibration einer Meßflüssigkeit mit dem Blut erreicht, welche anschließend auf die interessierende Substanz analysiert wird.

Bei den auf einer Seite von der Meßflüssigkeit und auf der anderen Seite von der Körperflüssigkeit, beispielsweise von Blut, beaufschlagten Membranen, welche für eine ausreichende Equilibration genügend groß sein müssen, kommt es jedoch ebenfalls bald zu Verunreinigungen bzw. zu einer Verstopfung der Membranen durch Körpersubstanzen, sodaß der Austausch der interessierenden Stoffe rasch behindert wird. Bei den in den Blutstrom eingebrachten Fremdkörpern, wie Kanülen und Kathetern, besteht zudem die ständige Gefahr der Infektion, der Thrombose oder der Embolie. Außerdem stehen für über längere Zeit aufrecht zu erhaltende Therapien nur wenige Blutgefäße zur Verfügung, welche bald thrombosieren, sodaß das Verfahren nicht fortgesetzt werden kann.

Aufgrund der erwähnten Schwierigkeiten gibt es derzeit auch keine zufriedenstellende Vorrichtung, welche sich zur laufenden, ambulanten Kontrolle, z.B. des Gewebezuckers und der Korrektur desselben, eignet.

Aufgabe der Erfindung ist es nun, eine einfache, möglichst auch vom Patienten anwendbare, kompakte bzw. tragbare Vorrichtung vorzuschlagen, mit welcher die Bestimmung der Konzentration von im lebenden Organismus vorliegenden Substanzen möglich ist, ohne daß dabei die Gefahr von Infektionen, Thrombosen oder Embolien gegeben ist.

Diese Aufgabe wird erfindungsgemäß dadurch gelöst, daß die als Subcutannadel/-katheter ausgeführte Hohlnadel in das Gewebe des Organismus einbringbar ist und einen in Wandbereichen offenen Austauschkanal aufweist, daß die Einrichtung zur Zufuhr der Perfusionsflüssigkeit und zur Abfuhr nach deren teilweisen Equilibration mit der zu bestimmenden Substanz, vorzugsweise der Glucose, und im Vergleich zur zu bestimmenden Substanz relativ konstanten endogenen oder exogenen Markergrößen mit dem Austauschkanal verbunden ist, sowie daß eine mit der Zu- und Abfuhreinrichtung verbundene Analyseneinrichtung zur Messung der Konzentration der Substanz und einer Markergröße vorhanden ist, welche an die Auswerteeinheit angeschlossen ist, welche bei teilweiser Equilibration die im Gewebe vorliegende Konzentration der zu bestimmenden Substanz ermittelt. Mit dieser Vorrichtung wird eine Perfusionsflüssigkeit direkt in das Gewebe eingebracht. Die Perfusionsflüssigkeit wird nach deren teilweisen Equilibration mit der zu bestimmenden Substanz des Gewebes gesammelt und auf die zu bestimmende Substanz sowie auf endogene oder exogene Markergrößen analysiert. Der Grad der Wechselwirkung der Perfusionsflüssigkeit mit dem Gewebe wird durch die Markergröße angezeigt. Die im Gewebe vorliegende Konzentration der zu bestimmenden Substanz kann dann mit Hilfe der Markergrößen bestimmt werden. Zum Unterschied von herkömmlichen Vorrichtungen, welche ausschließlich in Blutgefäßen oder anderen flüssigkeitsgefüllten Körperhöhlen angewandt werden, wird durch die erfindungsgemäße Vorrichtung im Gewebe, d.h. im geschlossenen Zellverband, ein vorher nicht vorhandener Hohlraum geschaffen, in welchem die in das Gewebe eingebrachte Perfusionsflüssigkeit direkt, d.h. ohne Zwischenlage irgendwelcher Membranen, mit dem Gewebe des Organismus in Wechselwirkung tritt. Vorteilhafterweise ist dabei nur eine teilweise Equilibration notwendig, da die Perfusionsflüssigkeit sowohl auf die interessierende Substanz, als auch auf endogene oder exogene Markergrößen analysiert wird, welche den Grad der Wechselwirkung der Perfusionsflüssigkeit mit dem Gewebe anzeigen. Die Vorteile der neuen Vorrichtung liegen auf der Hand. Durch die Verlagerung des Meßortes ins Gewebe, werden Thrombosen und Embolien vollkommen ausgeschaltet, sowie das Risiko einer Infektion stark vermindert. Ein weiterer Vorteil ist dadurch gegeben, daß man für die Messung nicht auf einige, wenige zugängliche Blutgefäße angewiesen ist.

Als endogene Markergröße der Perfusionsflüssigkeit kann zumindest ein Parameter aus der Gruppe Ionenkonzentration, Leitfähigkeit, Impedanz, Osmolarität und Dichte analysiert werden. Zur Messung einer exogenen Markergröße kann der Perfusionsflüssigkeit zumindest eine auf chemischem oder physikalischem Wege erfaßbare Substanz zugesetzt werden. Damit kann aus der gemessenen und der ursprünglich bekannten Quantität der Markergröße der Grad der Equilibration der eingebrachten Perfusionsflüssigkeit errechnet werden, sodaß aus der gemessenen Konzentration der interessierenden Substanz und aus dem Grad der Verdünnung der eingebrachten Perfusionsflüssigkeit die tatsächlich im Gewebe vorliegende Konzentration der zu bestimmenden Substanz bestimmt werden kann.

Die sich im Körper befindliche Nadel der Vorrichtung weist somit an ihrer Oberfläche einen Kanal auf, dessen Wände zumindest zum Teil direkt vom Gewebe gebildet werden, sodaß die Perfusionsflüssigkeit ungehindert an das Gewebe herantreten kann, wo teilweise Equilibration eintritt und anschließend gesammelt und analysiert werden kann. Die beschriebene Vorrichtung kann vom Patienten insofern problemlos gehandhabt werden, als bei der Verwendung von Subcutannadeln oder Subcutankathetern keinerlei Gefahr für eine Luftembolie oder eine Thrombose bestehen.

Weiters ist erfindungsgemäß vorgesehen, daß die Einrichtung zur Zu- und Abfuhr der Perfusionsflüssigkeit ein Reservoir für die Perfusionsflüssigkeit, eine zuführende Leitung zum Austauschkanal, eine über zumindest eine Öffnung mit dem Austauschkanal verbundene abführende Leitung zur Analyseneinrichtung, sowie ggf. einen über eine Leitung an die Analyseneinrichtung angeschlossenen Auffangbehälter für die Perfusionsflüssigkeit aufweist, wobei zumindest eine Druck- und/ oder Saugpumpe zur Umwälzung der Perfusionsflüssigkeit vorhanden ist, sowie daß eine Kalibriereinrichtung zur Kalibrierung der Sensoren in der Analyseneinrichtung vorhanden ist, welche einen Behälter für die Kalibrierlösung und eine durch Ventile steuerbare Verbindungsleitung zur Analyseneinrichtung, eine Pumpe für die Kalibrierlösung, sowie ggf. einen Auffangbehälter für die Kalibrierlösung aufweist. Damit kann die zur Analyse der interessierenden Substanz und der Markergröße vorhandene Analyseneinrichtung in vorgebbaren Zeitintervallen mit Hilfe eines Kalibriermediums kalibriert werden.

Aufgrund des von der Auswerteeinheit der Vorrichtung errechneten Wertes für die Konzentration der zu bestimmenden Substanz kann die Dosis der zu verabreichenden Medikamente bzw. Art und Umfang der notwendigen Therapie auch über lange Zeiträume exakt auf den Patienten abgestimmt werden.

Erfindungsgemäß ist es da von Vorteil, wenn eine Einrichtung zur Zufuhr eines Medikamentes vorhanden ist, welche ein Reservoir für das Medikament, eine Medikamentenpumpe und eine Zuleitung für das Medikament aufweist, wobei eine Medikamentenöffnung in der Zuleitung den Austritt des Medikaments ins Gewebe ermöglicht. Dabei wird in Abhängigkeit von der Konzentration der zu bestimmenden Substanz ein Medikament appliziert, wobei die Applikation vorzugsweise automatisch erfolgt.

Es kann beispielsweise die Glucosekonzentration und die endogene Natrium- bzw. Chloridkonzentration als Markergröße und ggf. die Temperatur der Perfusionsflüssigkeit bestimmt werden. Es ist natürlich auch möglich, die Glucosekonzentration als interessierende Meßgröße zu bestimmen und die Leitfähigkeit bzw. Impedanz der Perfusionsflüssigkeit als Markergröße heranzuziehen.

Will man sich jedoch auf eine exogene Markergröße stützen, kann z.B. die Glucosekonzentration und die Konzentration eines der Perfusionsflüssigkeit zugesetzten Farbstoffes als Markergröße bestimmt werden. Als exogene Markersubstanzen eignen sich alle körperverträglichen, im Körper nicht oder in geringer Menge vorkommenden Stoffe, wie eben Farbstoffe, deren Verdünnung photometrisch gemessen werden kann, Inulin, chemische oder auch radioaktive Substanzen. Beispielsweise würde die Berechnung der tatsächlichen Gewebeglucose bei Verwendung des Gewebenatriums als Marker, das in einer konstanten Konzentration von 140 mmol/l vorliegt, bei einem in der wiedergewonnenen Perfusionsflüssigkeit gemessenen Wert von z.B. 14 mmol/l $Na^+$ eine Multiplikation der gemessenen Glucosekonzentration mit einem Faktor 10 erfordern.

Bei Verwendung der Ionenkonzentration als Markergröße ist es von Vorteil, wenn als Perfusionsflüssigkeit eine glucosearme, ionenarme Lösung verwendet wird, welche ggf. annähernd isoton ist.

Beispiele für die Anwendung der erfindungsgemäßen Vorrichtung sind vielfältig. Wie bereits erwähnt, kann eine künstliche Bauchspeicheldrüse zur Kontrolle des Diabetes mellitus realisiert werden, oder eine Vorrichtung zur Erfassung und Beseitigung anderer Hormonstörungen, wie z.B. im Zusammenhang mit dem Calziumstoffwechsel, Wachstumshormon, Geschlechtshormonen, u.s.w.

Da man die Konzentration der Substanzen in der Kalibrierlösung so halten kann, daß sie in der Größenordnung der in der Perfusionsflüssigkei zu erwartenden Konzentrationen der interessierenden Substanz liegen, ist die Linearität der Analyseneinrichtung im interessierenden Bereich ggf. bereits durch eine Einpunktskalibrierung gewährleistet. Falls diese Kalibrierung nicht ausreicht, müßte in einer zweiten Kalibrierung z.B. der Nullpunkt bestimmt werden.

Im konkreten Beispiel der Anwendung in einer künstlichen Bauchspeicheldrüse wird man z.B. Glucose und Natrium-Chlorid der Kalibrierlösung zusetzen, wobei sich eine Konzentration von ca. 5 bis 40 mg/dl bzw. von 5 bis 40 mmol/l eignen würde. Zusätzlich besteht auch die Möglichkeit, den Nullpunkt auf einfache Weise nachzukalibrieren, wenn die Perfusionsflüssigkeit frei von bzw. arm an der zu messenden Substanz und Markersubstanzen ist, und intermittierend ohne Gewebekontakt an die Analyseneinrichtung gebracht werden kann.

In einer Weiterbildung der Erfindung ist vorgesehen, daß die Subcutannadel/-katheter zumindest zwei Leitungen aufweist, daß eine der Leitungen als mit Öffnungen in Wandbereichen ausgestatteter Austauschkanal und die andere als mit diesen über zumindest eine Öffnung im Bereich der Spitze der Subcutannadel/-katheter verbundene abführende Leitungen für die Perfusionsflüssigkeit ausgebildet ist, wobei ein Großteil der Nadeloberfläche als Equilibrationsfläche zur Verfügung steht. Über die in den Körper eingebrachte Subcutannadel, die mit der zuführenden und der abführenden Leitung der übrigen Vorrichtung in Verbindung steht, wird mit einer Pump- und/oder Saugvorrichtung Perfusionsflüssigkeit in den Körper eingebracht und wieder abgeführt, wobei sich das Gewebe am Austauschkanal bzw. an der Nadeloberfläche zwar abstützt aber nicht in den Kanal eindringen kann. Es entsteht dadurch an der Oberfläche der Subcutannadel bzw. des Subcutankatheters eine ausreichend große Fläche, welche der Equilibration der Perfusionsflüssigkeit mit dem umliegenden Gewebe zur Verfügung steht. In der mit der abführenden Leitung verbundenen Analyseneinrichtung sind zumindest zwei Sensoren bzw. Meßelektroden vorhanden, welche die zu bestimmende Substanz sowie die Markergröße erfassen.

Wenn eine Subcutannadel in den Körper eingebracht wird, kann es zur Verletzung von Körperzellen kommen, sodaß Enzyme, die z.B. Glucose spalten, freigesetzt werden können. Deshalb könnte es notwendig sein, Maßnahmen zu treffen, eine Umsetzung der zu bestimmenden Substanz, beispielsweise eine Glycolyse in der Perfusionsflüssigkeit zu verhindern. Zu diesem Zweck kann der Perfusionsflüssigkeit ein Substrat zugesetzt werden, das für seine Umsetzung Adenosintriphosphat benötigt. Beispielsweise käme 3-Phosphoglycerat in Frage, aus welchen bei Vorhandensein des notwendigen Enzyms 3-Phosphoglyceratkinase unter Verbrauch von ATP 1-3-Biphosphoglycerat entsteht. Da das erwähnte Enzym gleichzeitig mit den glycolytischen Enzymen freigesetzt wird, läßt sich so die Glycolyse und eine Verfälschung der gemessenen Glucosewerte verhindern. Das dabei entstehende 1-3-Biphosphoglycerat ist insoferne unkritisch als durch Mangel an weiteren Enzymen der Gluconeogenese in der Subcutis eine Neuentstehung von Glucose daraus unmöglich ist. Durch eine optimale Wahl der Konzentration von 3-Phosphoglycerat in der Perfusionsflüssigkeit muß man nur verhindern, daß die Reaktion durch das anfallende 1-3-Biphosphoglycerat wieder in Richtung Glycolyse gedrängt wird. Dafür würde sich z.B. eine Konzentration von 3-Phosphoglycerat in der Größenordnung von 5 bis 100 mmol/l anbieten.

Falls unterschiedliche Diffusionskonstanten vorliegen, ist es von Vorteil, daß zusätzlich die Diffusionskonstanten zumindest einer Markersubstanz und der zu bestimmenden Substanz in die Berechnung der Konzentration der zu bestimmenden Substanz eingehen.

In einer besonders einfachen Ausführungsvariante der Erfindung wird vorgeschlagen, daß die Subcutannadel/-katheter aus zwei ineinanderliegenden Kanülen mit unterschiedlichen Durchmessern besteht, wobei zwischen den beiden Kanülen ein Austauschkanal mit im wesentlichen ringförmigem Querschnitt entsteht und die innere Kanüle bis nahe an die Spitze der äußeren Kanüle reicht. In dieser zweilumigen Ausführung der Subcutannadel wird die innere Kanüle beispielsweise intermittierend zur Abfuhr der Perfusionsflüssigkeit und zur Zufuhr des Medikamentes verwendet. Es ist natürlich auch möglich, das Medikament anders, z.B. über eine weitere Injektionsnadel oder auch oral zu verabreichen. Aufgrund des möglichen geringen Außendurchmessers dieser Subcutannadeln im Bereich von ca. 0,5 mm wird die Schmerzbelastung des Patienten bei Anwendung der Vorrichtung auf ein Mindestmaß reduziert. Weitere erfindungsgemäße Ausführungsvarianten dieser zweilumigen Subcutannadeln bzw. Subcutankatheter sind im Detail in der Figurenbeschreibung dargestellt.

Falls man für die Zuleitung des Medikamentes einen separaten Kanal vorsehen will, ist erfindungsgemäß vorgesehen, daß die Zuleitung für das Medikament, die abführende Leitung und der Austauschkanal als im wesentlichen konzentrisch ineinanderliegende Kanülen ausgeführt sind, wobei die innerste Kanüle die Zuleitung für das Medikament ist und im Bereich der Spitze der Subcutannadel/-katheter eine Öffnung zum Gewebe aufweist, daß die äußere Kanüle über zahlreiche über einen möglichst großen Teil der Oberfläche verteilte Öffnungen verfügt, daß die abführende Leitung im Bereich der Spitze der Subcutannadel/-katheter zumindest eine Öffnung zum Austauschkanal aufweist und abführende Leitung sowie Austauschkanal im Bereich der Spitze ggf. blind verschlossen sind. Für weitere Ausführungsvarianten dreilumiger Subcutannadeln/-katheter wird wieder auf die Figurenbeschreibung verwiesen.

Für die Gestaltung der Bereiche, in welchen die Perfusionsflüssigkeit mit dem am Austauschkanal anliegenden Gewebe in Kontakt tritt, gibt es eine Reihe weiterer Möglichkeiten. Erfindungsgemäß ist beispielsweise vorgesehen, daß die Subcutannadel/-katheter eine innere Kanüle zur Aufnahme der abführenden Leitung und ggf. der Zuleitung für ein Medikament aufweist, wobei an der Oberfläche der Kanüle zahlreiche längs oder spiralig verlaufende Septen angeordnet sind, welche nach Einbringen der Kanüle in das Gewebe den Austauschkanal bilden, sowie daß zur Abfuhr der Perfusionsflüssigkeit die abführende Leitung zumindest eine Öffnung zum Raum zwischen den Septen aufweist, sowie daß die Subcutannadel/-katheter aus zum Teil elastischem Material besteht und zum Schutz der Septen in einer Hohlnadel untergebracht ist, welche nach dem Einstechen in das Gewebe entfernbar ist.

Durch die Verwendung einer Hohlnadel beim Einstechen kann die Subcutannadel bzw. der Subcutankatheter aus einem elastischen, gewebefreundlichen Material bestehen, welches relativ angenehm zu tragen ist. Zusätzlich können beispielsweise die elastischen Septen eines Subcutankatheters in der Hohlnadel umgelegt sein, so einen möglichst geringen Durchmesser für die einzustechende Hohlnadel aufweisen, und sich erst im Gewebe zur Bildung des Austauschkanals aufrichten.

Als weiterer Komfort für den Patienten ist vorgesehen, daß am von der Spitze abgewandten Ende der Subcutannadel/-katheter eine Steckverbindung angeordnet ist, welche den Austauschkanal bzw. die abführende Leitung in der Subcutannadel/-katheter mit der zuführenden Leitung bzw. mit den außerhalb der Nadel liegenden Teil der abführenden Leitung verbindet, wobei aufgrund der geometrischen Konfiguration der Steckverbindung eine eindeutige Zuordnung der in der Subcutannadel/-katheter vorliegenden Leitungen zum übrigen Teil der Vorrichtung möglich ist.

Aufgrund der geforderten Kompaktheit eines tragbaren Gerätes ist in einer vorteilhaften Weiterbildung vorgesehen, daß die Druck- und Saugpumpe der Einrichtung zur Zu- und Abfuhr der Perfusionsflüssigkeit durch eine Kolbenpumpe realisiert ist, wobei der an beiden Seiten abgeschlossene Kolbenraum einen durch den Kolben vom Reservoir getrennten Auffangbehälter für die Perfusionsflüssigkeit bildet, wobei der Antriebsstempel der Kolbenpumpe gegen die Außenwand der Kolbenpumpe abgedichtet ist. Die genannte Lösung gilt natürlich nicht nur für die Perfusionsflüssigkeit, sondern auch z.B. für die Kalibrierlösung, für welche ebenfalls mittels einer Kolbenpumpe auf platzsparende Weise das Reservoir und der Auffangbehälter für die Kalibrierlösung in ein Pumpgerät vereinigt werden können, sodaß anstelle von Druck und Saugpumpen nur eine Kolbenpumpe mit einem Antrieb notwendig ist. Insgesamt wären somit in einer kompakten Ausführung der erfindungsgemäßen Vorrichtung für die Perfusionsflüssigkeit, für die Kalibrierlösung und das Medikament nur jeweils eine Kolbenpumpe notwendig.

Eine kompakte, konstruktiv einfache und robuste Ausführung der Vorrichtung ist dadurch gegeben, daß alle Komponenten der Vorrichtung und deren Energieversorgung in einem implantierbaren Gehäuse angeordnet sind, daß die Oberfläche des Gehäuses zumindest zum Teil als in Wandbereichen offener Austauschkanal ausgebildet ist, daß die Einrichtung zur Zufuhr der Perfusionsflüssigkeit und zur Abfuhr nach deren teilweisen Equilibration mit der zu bestimmenden Substanz, vorzugsweise der Glucose, und im Vergleich zur zu bestimmenden Substanz relativ konstanten endogenen oder exogenen Markergrößen mit dem Austauschkanal verbunden ist, sowie daß eine mit der Zu- und Abfuhreinrichtung verbundene Analyseneinrichtung zur Messung der Konzentration der Substanz und einer Markergröße vorhanden ist, welche an die Auswerteeinheit angeschlossen ist, welche bei teilweiser Equilibration die im Gewebe vorliegende Konzentration der zu bestimmenden Substanz ermittelt, sowie daß ein Reservoir für die Perfusionsflüssigkeit und ein Reservoir für ein Medikament je durch ein Septum transcutan nachfüllbar sind. Im Fall eines implantierbaren Gerätes, beispielsweise einer implantierbaren Insulinpumpe, könnte z.B. ein Teil der Oberfläche des Gerätes bzw. die gesamte Oberfläche einen z.B. mäanderförmig angelegten Austauschkanal aufweisen, welcher mit seinen Öffnungen zum Gewebe eine riesige Equilibrationsfläche darstellt. Aufgrund der geringen Perfusionsraten könnte bei einem implantierbaren Gerät auch der Auffangbehälter für das Perfusat entfallen, da die Perfusionsflüssigkeit nach deren Analyse in den Körper abgeleitet und dort resorbiert werden könnte. Gleiches wäre auch für die Kalibrierlösung denkbar.

Aufgrund der geforderten Sicherheit bei implantierbaren Geräten ist es von Vorteil, wenn die zu bestimmende Substanz und die Markergröße von zumindest zwei unabhängigen Analyseneinrichtungen erfaßt werden, wobei bei Nichtübereinstimmung der Meßwerte Alarm gegeben wird.

Obwohl eine implantierte Vorrichtung nach der Erfindung gewisse Vorteile aufweist, ergeben sich auch für jene Ausführungsvarianten, bei welchen lediglich eine Subcutannadel/-katheter ins Körpergewebe eingebracht wird, positive Aspekte. Beispielsweise können optimale Bedingungen für die Messung eingehalten werden. Dies betrifft z.B. die Temperatur, bzw. deren Konstanthaltung oder Eingang derselben in die Berechnung und den pH-Wert der Perfusionsflüssigkeit, wobei mit pH = 5,6 eine optimale Immobilisierung des Enzyms Glucoseoxidase erreicht wird. Bei einer Perfusionsrate zwischen ca. 1 $\mu$l und 10 $\mu$l/min kann bei der erfindungsgemäßen Vorrichtung eine Verdünnung der im Gewebe herrschenden Glucosekonzentration um einen Faktor von 3 bis 40 erreicht werden, was eine optimale Glucosekonzentration für die Messung darstellt und hilft, einen zu raschen Verbrauch der glucosespaltenden Enzyme und eine Sauerstoffdepletion an der Elektrode zu vermeiden. Darüber hinaus könnte auch die Perfusionsflüssigkeit mit Sauerstoff angereichert werden. Bei Verwendung von elektroaktiven Meßeinrichtungen ist die räumliche Trennung der Analyseneinrichtung vom Körper auch insoferne von Vorteil, als im Körper keine katalytischen Produkte freiwerden können.

Wenn Patienten für sie wichtige medizinische Parameter, wie z.B. ihre Zuckerwerte regelmäßig kontrollieren müssen, ist es für sie gegenüber umständlichen und schmerzhaften herkömmlichen Verfahren und Vorrichtungen, z.B. dem Anstechen der Fingerkuppen zur Gewinnung von Blut, von Vorteil, wenn ihnen ein schreibstift- oder handgriffähnliches Gehäuse zur Verfügung steht, an welchem die Subcutannadel/-katheter befestigbar ist, wobei das schreibstiftähnliche Gehäuse die Einrichtung zur Zu- und Abfuhr einer Perfusionsflüssigkeit, die damit verbundene Analyseneinrichtung und die Auswerteeinheit aufweist, wobei für die Darstellung der gemessenen Parameter eine an die Auswerteeinheit angeschlossene Anzeige vorhanden ist. Die Medikamentenzufuhr, beispielsweise die nötige Insulinmenge, kann dann vom Patienten aufgrund dieser Anzeige bestimmt und selbsttätig zugeführt werden.

In einer weiteren komfortableren Ausgestaltung der Erfindung ist vorgesehen, daß im Gehäuse zusätzlich eine Einrichtung zur Zufuhr eines Medikamentes vorhanden ist, wobei die Medikamentenapplikation durch die bereits vorhandene Subcutannadel/-katheter erfolgt. So kann beispielsweise ein insulinpflichtiger Patient mit nur einem Einstich der erfindungsgemäßen Subcutannadel den Glucosewert bestimmen und durch diese auch die erforderliche Medikamentenmenge verabreichen.

Eine besonders einfache, erfindungsgemäße Vorrichtung sieht dabei vor, daß die Kolbenpumpe für die Perfusionsflüssigkeit, die Medikamentenpumpe und ggf. die Pumpe für die Kalibrierflüssigkeit, über eine Hebeleinrichtung manuell betätigbar sind.

Wenn die schreibstiftähnliche Vorrichtung, beispielsweise in einer Jackentasche oder Handtasche mitgeführt wird, kann man über die Nadel bzw. bei abgenommener Nadel, über das Nadelansatzstück eine eng abdichtende Hülle bzw. Abdeckung anbringen, die für Sterilität sorgt, keine Verdunstung der Perfusionsflüssigkeit zuläßt und bei der Betätigung der Perfusionspumpe einen Rückfluß der unverbrauchten Perfusionsflüssigkeit in die Analyseneinrichtung ermöglicht. So kann man z.B. mit der Perfusionsflüssigkeit, welche im unbenutzten Zustand frei von bzw. arm an den zu messenden Substanzen ist, die Meßkapillare der Analyseneinrichtung benetzen, um zu verhindern, daß die Sensoren und Meßelektroden durch Austrocknen beschädigt werden. Man könnte der Perfusionsflüssigkeit auch spezielle Zusätze beifügen, welche die Schonung der Analyseneinrichtung in noch besserer Weise garantieren würden. Als solche Zusätze kämen Substanzen in Frage, die z.B. bei Verwendung von immobilisierter Glucoseoxidase oder einem anderen Enzym den Gehalt an immobilisiertem Enzym weiter verbessern würden. Die Perfusionsflüssigkeit kann dabei auch zur Nullpunktseichung vor der nächsten Messung am Patienten herangezogen werden.

Sowohl bei der Ausführung der schreibstiftähnlichen Vorrichtung als Meßvorrichtung, beispielsweise eines "Glucose-Pen" als auch bei der Ausführung als kombiniertes Meß- und Therapiegerät, beispielsweise eines "Glucose-Insulin-Pen" als auch bei der implantierbaren oder tragbaren Vorrichtung, ist es im Sinne der Miniaturisierung der einzelnen Vorrichtungskomponenten von Vorteil, wenn die zur Zuschaltung der einzelnen Zu- und Abführleitungen zu den einzelnen Behältern benötigten Ventile durch ein Mehrwegventil realisiert sind, dessen Ventilkörper alle Zu- und Abführleitungen enthält und dessen beweglicher Teil Kanäle bzw. Nuten zu deren eindeutigen Zuordnung aufweist, wobei einzelne Positionen des beweglichen Teils durch ein Stellrad oder automatisch durch an einer Antriebseinheit anliegende Signale der Auswerteeinheit einstellbar sind. Durch ein Mehrwegventil sind nicht nur alle in der Figurenbeschreibung ausführlich beschriebenen, notwendigen Zuordnungen von Leitungen und Behältern möglich, sondern es können auch viele oder alle notwendigen Schaltvorgänge für die einzelnen Pumpen über den beweglichen Teil des Mehrwegventils realisiert werden.

Als wichtigste Stellungen dieses Mehrwegventils seien hier folgende genannt:

1. ein Meßbetrieb, bei welchem der Austauschkanal der Vorrichtung mit der Zu- und Abfuhreinrichtung für die Perfusionsflüssigkeit, sowie diese mit der Analyseneinrichtung und ggf. die Medikamentenleitung mit der Medikamentenzufuhr in Verbindung steht,

2. ein Kalibrierbetrieb (Steilheit), bei welchem die Kalibrierlösung der Analyseneinrichtung zugeführt wird,

3. ein Kalibrierbetrieb (Nullpunktskalibrierung), bei welchem der Analyseneinrichtung frische Perfusionsflüssigkeit zugeführt werden kann,

4. eine Stellung zum Zuführen von Luft um die Perfusionsflüssigkeit von der Kalibrierlösung zu trennen,

5. eine Stellung, in welcher in die Perfusionsstrecke Luftblasen eingebracht werden können, um das Perfusionsflüssigkeit zeitlich zu fraktionieren bzw.

6. eine Stellung, in welcher die Kalibrierlösung in die Medikamentenleitung eingebracht werden kann, um diese auch dann frei zuhalten, wenn längere Zeit kein Medikament verabreicht wird oder sich in der Kalibrierlösung eine zum Medikament antagonistische Substanz befindet.

Zur Aufnahme für die in den Betriebsstellungen 4 bzw. 5 zugeführte Luft kann es von Vorteil sein, wenn in der Vorrichtung ein zusätzlicher Auffangbehälter vorhanden ist. Es würde sich dafür beispielsweise der in der Medikamentenpumpe freiwerdende Kolbenraum eignen.

In einer weiteren Ausgestaltung der Erfindung kann auch vorgesehen sein, daß in der abführenden Leitung in der zum Auffangbehälter führenden Leitung oder im Auffangbehälter für die Perfusionsflüssigkeit ein Unterdrucksensor vorgesehen ist, sowie daß in der Analyseneinrichtung ein Temperatursensor vorgesehen ist. Bei einer allfälligen Verstopfung der Perfusionsstrecke ist über den Unterdrucksensor ein Ab- bzw. Umschalten der Druck- und Saugpumpen für das Perfusat möglich. Dieser Unterdrucksensor ist vorallem insoferne von Vorteil, als im Auffangbehälter beim Meßbetrieb ein deutlicher Unterdruck herrscht, sodaß beim Umschalten auf die Zufuhr von Luft zuviel Luft angesaugt und damit der Auffangraum vergeudet würde. Abgeholfen könnte dem durch ein kurzes gegensinniges Laufen der Kolbenpumpe vor dem Umschalten des Mehrwegeventils werden.

Weiters ist es erfindungsgemäß von Vorteil, daß die Analyseneinrichtung zur Erfassung der zu messenden Substanz und der endogenen oder exogenen Markergrößen als austauschbarer Block vorliegt, bei dessen Ausbau die mit der Analyseneinrichtung in Verbindung stehenden Leitungen über einen Verschließmechanismus abschließbar sind, sodaß der aufgebaute Druck in diesen Leitungen erhalten bleibt, wobei als weiterer Vorteil anzuführen ist, daß alle Behälter und Reservoire sowie alle zu- und abführenden Leitungen als Einmal-System auf einem gemeinsamen Träger untergebracht sind, wobei durch das Einsetzen dieses gemeinsamen Trägers in die Vorrichtung der Kraftschluß zwischen den Antrieben für die Pumpen und für das Mehrwegventil automatisch herstellbar ist. Alle Behälter und Reservoire können so auf einfache Weise mit einem Handgriff durch ein neues Set ersetzt werden.

Schließlich ist in einer weiteren erfindungsgemäßen Ausführungsvariante daran gedacht, daß ein Dosierschalter zur Steuerung der Medikamentendosis vorhanden ist, in dessen ersten Stellung die Dosierung automatisch und kontinuierlich in der von der Auswerteeinheit errechneten Weise erfolgt und in dessen zweiten Stellung eine vom Benutzer vorbestimmte Medikamentendosis applizierbar ist. Damit wird es dem Benützer auch möglich, sich eine mehrere Stunden wirkende Bolusgabe des Medikamentes zu verabreichen. Der Mikroprozessor der Auswerteeinheit könnte dabei dem Patienten helfen, die richtige Bolusgröße zu finden.

Die Erfindung wird nachstehend anhand von Ausführungsbeispielen näher erläutert. Es zeigen in zum Teil schematischer Darstellung:

Fig. 1 eine Vorrichtung nach der Erfindung,

Fig. 2 einen Schnitt im Bereich der Subcutannadel bzw. des Subcutankatherers nach der Linie II-II in Fig. 1,

Fig. 3 bis 6 Ausführungsvarianten in einer Fig. 2 entsprechenden Schnittdarstellung,

Fig. 7a bis 7f Längs- und Querschnitte weiterer Ausführungsvarianten der Subcutannadel/-katheter,

Fig. 7g ein Detail aus Fig. 7,

Fig. 8 bis 11 Ausführungsvarianten einer Pumpvorrichtung nach Fig. 1,

Fig. 12 eine weitere Ausführungsvariante nach Fig. 1,

Fig. 13a bis 13d Ventilstellungen eines Mehrwegventils nach Fig. 12,

Fig. 14 ein Detail aus Fig. 12,

Fig. 15 ein Detail einer implantierbaren Vorrichtung,

Fig. 16 eine Ausführungsvariante nach Fig. 1 und 12 als "Glucose-Pen",

Fig. 17 bis 19 Ausführungsvarianten nach Fig. 1 und 12 als "Glucose-Insulin-Pen",

Fig. 20 eine Kalibriervorrichtung für die Ausführungen nach Fig. 16 bis 19 sowie die

Fig. 21 und 22 experimentell ermittelte Meßkurven.

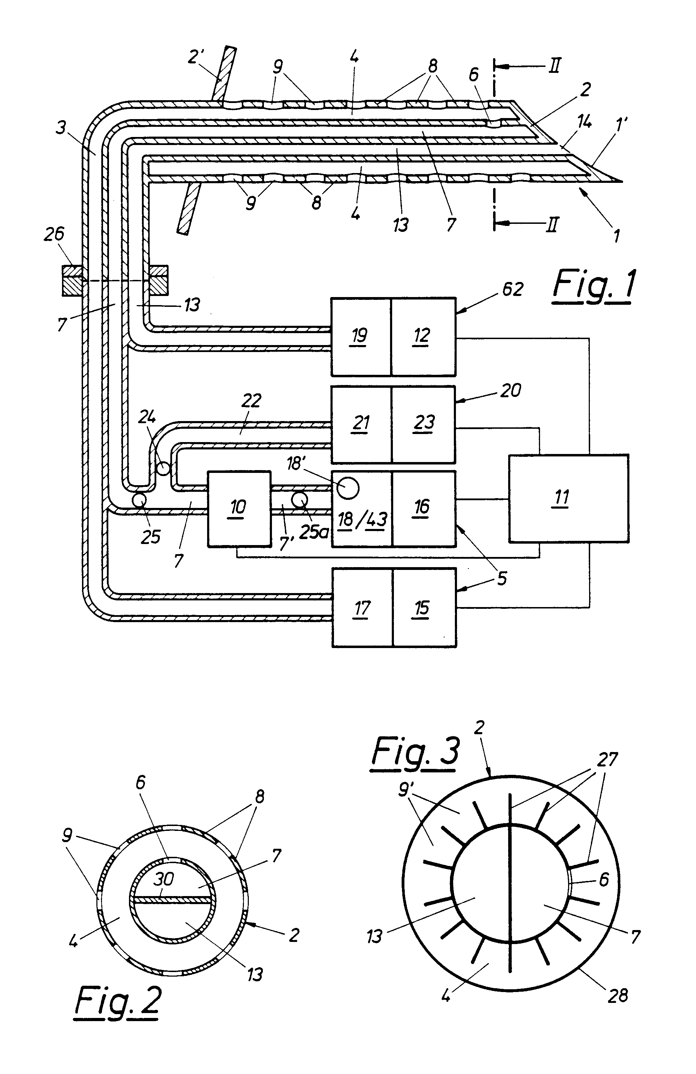

Eine Vorrichtung zur Bestimmung der Konzentration von Substanzen im Gewebe lebender Organismen ist schematisch in Fig. 1 dargestellt. Der ins Gewebe einbringbare Teil 1 ist hier als Subcutannadel oder Subcutankatheter 2 ausgeführt.

In der Subcutannadel 2 verläuft ein durch Öffnungen 9 mit dem umgebenden Gewebe in direktem Kontakt stehender Austauschkanal 4, der über eine zuführende Leitung 3 mit der Zu- und Abfuhreinrichtung 5 für die Perfusionsflüssigkeit verbunden ist. Nach der zumindest teilweisen Equilibration mit dem Gewebe gelangt die Perfusionsflüssigkeit über eine Öffnung 6 im Bereich der Spitze 1' der Subcutannadel 2 in eine abführende Leitung 7 und wird durch diese einer Analyseneinrichtung 10 zugeführt. Brückenförmige Überdachungen 8 zwischen den Öffnungen 9 verhindern ein Eindringen des Gewebes in den Austauschkanal 4.

Die Analyseneinrichtung 10 weist beispielsweise einen Zweikanal-Analysator, z.B. für Glucose als interessierende Substanz und die Gleichstrom- oder Wechselstromleitfähigkeit als Markergröße auf, wobei der Rechner der angeschlossenen Auswerteeinheit 11 aus der errechneten Gewebszuckerkonzentration eine Insulin- oder andere Medikamentenpumpe 12 so steuert, daß Normoglykämie erreicht wird. Das Insulin gelangt dabei z.B. über eine Zuleitung 13 an die Spitze 1' der Nadel oder des Katheters, um dort durch zumindest eine Medikamentenöffnung 14 ins Gewebe auszutreten. Aus Gründen der Vereinfachung kann eine gemeinsame Pumpvorrichtung, wie z.B. Rollenpumpe, Peristaltikpumpe, Druckbehälter oder ähnliches eingesetzt werde, um die Druckpumpe 15, die Saugpumpe 16 für die Perfusionsflüssigkeit und die Medikamentenpumpe 12 zu verwirklichen. Für die Perfusionsflüssigkeit steht in der Zu- und Abfuhreinrichtung ein Reservoir 17 sowie ein Auffangbehälter 18 zur Verfügung, in der Einrichtung 62 zur Zufuhr eines Medikamentes ein Medikamentenreservoir 19. Der Rechner der Auswerteeinheit 11 kann auch dazu verwendet werden, um aus der Konzentration der Markersubstanz die optimale Arbeitsrate der Druckpumpe 15 und Saugpumpe 16 für die Perfusionsflüssigkeit zu steuern. Bei optimaler geometrischer Konfiguration könnte es möglich sein, die Perfusionsflüssigkeit ausschließlich mit der Saugpumpe 16 durch alle Leitungen zu transportieren, während der Transport mit der Druckpumpe 15 allein problematisch zu sein scheint, da die Flüssigkeit bei Überdruck in zu großem Maß durch die Öffnungen 9 ins Gewebe austreten würde. Die Zusammensetzung der Perfusionsflüssigkeit kann so optimiert werden, daß der Stoffaustausch über die Öffnungen 9 verbessert wird, so kann z.B. eine hyperosmotische oder hyperonkotische Flüssigkeit verwendet werden oder z.B. Stoffe, die die Gerinnung hemmen, wie z.B. Heparin oder ein Gewebsplasminogen-Aktivator zugesetzt werden. Besonders haben sich in Versuchen einfache Lösungen wie ionenarmes Wasser oder eine ca. 5%ige Mannitlösung mit Zusatz von Heparin bewährt. Mit Hilfe einer Kalibriereinrichtung 20, welche über die Verbindungsleitung 22 mit der abführenden Leitung 7 in Verbindung steht, kann eine Kalibrierlösung von einem Behälter 21 mittels einer Pumpe 23 an die Sensoren der Analyseneinheit 10 herangebracht werden, sodaß diese z.B. vor jeder Messung nachkalibriert werden können.

Bei stabilen Sensoren, bzw. Meßelektroden, könnte die Leitung 22 der Behälter 21 mit der Kalibrierlösung und die Pumpe 23 selbstverständlich entfallen, bzw. wäre es auch denkbar, die Sensoren und Meßelektroden der Analyseneinrichtung 10, speziell wenn diese leicht austauschbar ist, außerhalb der Vorrichtung zu überprüfen.

Die Pumpe 23 für die Kalibrierlösung, die Druckpumpe 15 für die Perfusionsflüssigkeit, die Saugpumpe 16 für die wiederzugewinnende Perfusionsflüssigkeit, sowie die Pumpe 12 für das Medikament können im wesentlichen gleichartig aufgebaut sein, da für alle Pumpen ähnliche Pumpraten, in der Größenordnung von Mikroliter pro Minute, benötigt werden. Die Verbindungsleitung 22 könnte z.B. auch auf der anderen Seite der Analyseneinrichtung 10 zwischen dieser und dem Auffangbehälter 18 für die Perfusionsflüssigkeit in die Leitung 7' münden, wobei die Perfusionsrichtung in der Analyseneinrichtung 10 dann umgedreht wäre. Um ein Vermischen der Perfusionsflüssigkeit mit der Kalibrierlösung zu verhindern, ist es vorteilhaft, die abführende Leitung 7 von der Verbindungsleitung 22 bzw. die Analyseneinrichtung 10 vom Auffangbehälter durch ein oder mehrere Ventile 24, 25, 25a bzw. durch ein Mehrwegventil zu trennen. Dabei könne es sich z.B. um Ventile handeln, die ausschließlich durch die Druckverhältnisse in den Leitungen 7, 7' und 22 gesteuert werden, nämlich so, daß bei Druckanstieg in der Leitung 22, die abführende Leitung 7 verschlossen wäre, sodaß die Kalibrierlösung zwar an die Analyseneinrichtung 10 gelangen, jedoch keine Vermischung mit der Perfusionsflüssigkeit in der Leitung 7 stattfinden kann. Umgekehrt würde bei Perfusion der Analyseneinrichtung 10 mit Perfusionsflüssigkeit aus der Leitung 7, die Ventile 24, 25 so gestellt sein, daß aus der Leitung 22 keine Kalibrierlösung an die Analyseneinrichtung 10 kommen kann. Statt der durch die Druckverhältnisse gesteuerten Ventile, welche innerhalb der Leitungen durch den Strom der Flüssigkeit in den Ventilsitz gepreßt werden, könnten auch andere Ventile, z.B. Magnetventile vorhanden sein, die die Leitungen 22, 7, 7' von außen abklemmen oder öffnen, wofür sich gut weiche, komprimierbare Leitungen 7, 22, bzw. 7' eignen würden, wobei je nach eingeschalteter Pumpvorrichtungen das entsprechende Ventil vom Signal des Rechners in der Auswerteeinheit 11 geöffnet oder geschlossen werden könnte. Gleichgültig ob für diesen Zweck ein Mehrwegventil oder Ventile 24, 25, 25a verwendet werden, sollte gewährleistet sein, daß nur für den Umschaltvorgang, jedoch nicht für den eingestellten Zustand der Ventile bzw. des Mehrwegventils Energie verbraucht wird.

In die Leitungen 7, 7' oder auch den Auffangbehälter 18 sollte eventuell ein Unterdrucksensor 18' angebracht sein, der meldet, wenn das Perfusionssystem an irgend einer Stelle verstopft ist, bzw. der eventuell veranlaßt, daß die Verstopfung durch Umkehr des Flüssigkeitsstroms im Perfusionssystem beseitigt wird.

Das gesamte Behältersystem, bestehend aus den Behältern 17, 18, 21 und zusätzlich eventuell auch 19, sowie 43, falls ein eigener Auffangbehälter für die Kalibrierlösung vorhanden ist, sollte am besten so ausgeführt sein, daß das komplette System gleichzeitig als Set mit den Leitungen 3, 7, 7', 13 und 22 ausgetauscht werden kann. Damit wäre eine einfache Handhabung durch den Patienten mit Ausschluß des Verwechselns von einzelnen Behältern oder Leitungen gewährleistet. Um das Verbleiben der Subcutannadel bzw. des Subcutankatheters 2 im Körper auch bei komplettem Austausch des Behälter- und Leitungssystems zu gewährleisten, ist eine gemeinsame Steckverbindung 26 für die Leitungen 3, 7 und 13 vorgesehen, an welcher das ausgetauschte System wieder mit der Subcutannadel bzw. dem Subcutankatheter 2 verbunden werden kann. Durch eine entsprechende geometrische Konfiguration der Steckverbindung ist gewährleistet, daß bei Handhabung durch den Patienten die einzelnen Leitungen nicht miteinander verwechselt werden können. Mit einer Schraubsicherung, beispielsweise einer Überwurfmutter, könnte die gemeinsame Steckverbindung 26 gesichert werden. Die Analyseneinrichtung 10 kann bei Verwirklichung eines künstlichen Pankreas eine Glucoseelektrode, kombiniert mit einer ionenselektiven Elektrode oder einem Leitfähigkeitsmesser, aufweisen. Dies hätte den Vorteil, daß für beide Messungen die selbe Elektronik und eventuell auch zumindest teilweise die selben Elektroden herangezogen werden könnten.

In der Analyseneinrichtung 10 könnten sehr wohl auch konventionelle chemische Methoden angewandt werden, speziell dann, wenn das notwendige Reaktionsgemisch vor der Analyse der Perfusionslösung zugesetzt wird und statt der Elektroden beispielsweise ein Photometer verwendet wird, was besonders auch bei trockenchemischen Messungen in Frage käme.

Obwohl es denkbar wäre, das Medikament zur Beseitigung der im Organismus festgestellten Störung auch der Perfusionsflüssigkeit im Reservoir 17 zuzusetzen, ist es vom Medikamentenverbrauch her ökonomischer dafür ein eigenes Reservoir 19 und die zusätzliche Zuleitung 13 zu verwenden. Man wird vorteilhafter Weise auch den Abstand zwischen den Öffnungen 9 und der Medikamentenöffnung 14 in der Subcutannadel 2 so wählen, daß möglichst wenig Medikament wieder durch die abgepumpte Perfusionsflüssigkeit verloren geht. Um einen Verlust von eingebrachtem Medikament durch die abführende Leitung 7 zu verhindern, ist z.B. die zuführende Leitung 3 bzw. der Austauschkanal 4 sowie die abführende Leitung 7 an der Spitze 1' des Katheters oder der Nadel 2 blind verschlossen.

Damit mit der abführenden Leitung 7 bei zu großem Unterdruck nicht auch Luft an der Einstichstelle angesaugt wird, bewährt es sich, die Perfusionsrichtung von der Körperoberfläche zur Körpertiefe zu wählen, wie das in Fig. 1 dargestellt ist. Ein Ansaugen von Luft durch die Leitung 7 würde allerdings vom Leitfähigkeitsmesser oder Ionensensor in der Analyseneinrichtung 10 dedektiert werden, welche nicht nur Alarm geben, sondern auch die notwendigen Maßnahmen, wie Reduzierung der Perfusionsrate, Umkehr der Pumprichtung u.s.w. einleiten könnte. Eine Abdichtungsplatte 2' an der Subcutannadel 2 könnte auch zusätzlich dafür sorgen, daß über die Hautöffnung keine Luft angesaugt werden kann. Zur störungsfreien Wiedergewinnung der Perfusionslösung günstig, die Druckpumpe 15 und die Saugpumpe 16 so zu gestalten, daß in der Zeiteinheit ähnliche oder gleiche Mengen von Perfusionsflüssigkeit in den Körper hineingepumpt und wieder abgesaugt werden, was sich bei einer gemeinsamen Ausführung der Pumpen 15 und 16 besonders einfach bewerkstelligen läßt.

Fig. 2 zeigt die beispielsweise Ausführung einer Subcutannadel/-katheter 2 nach einem Schnitt in Fig. 1. Mit 7 ist dabei die abführende Leitung vom Austauschkanal 4 gekennzeichnet, wobei die Öffnung zur abführenden Leitung 7 mit 6 bezeichnet ist. Zu einer maximalen Ausdehnung der Austauschfläche mit dem umliegenden Gewebe ist der Kanal 4 beispielsweise als Ringkanal ausgeführt und rund um die abführende Leitung 7 und die Medikamentenleitung 13 angebracht. Die brückenförmigen Überdachungen 8 des Kanals 4 begrenzen zahlreiche Öffnungen 9, die die Equilibrierfläche zum Gewebe darstellen.

Fig. 3 zeigt eine weitere Ausführungsform eines Katheters im Querschnitt, wobei der Austauschkanal 4 durch mehrere Septen 27 in der Längsrichtung des Katheters unterteilt wird. Die Teilung des Kanals 4 durch zahlreiche Septen 27, wenn möglich, in kapillare Spalten, hat den Vorteil, daß sich zwar das Gewebe an den Septen 27 abstützen und so nicht in die Perfusionsstrecke eindringen kann, daß aber trotzdem eine maximal große Austauschfläche in den Bereichen 9' zwischen den Septen 27 gewährleistet ist. Man könnte die Septen 27 auch gewindeförmig führen, um den Equilibrationsweg weiter zu verlängern. Der Katheter 2 wird, insbesondere wenn Teile oder der gesamte Katheter aus elastischem Material besteht, entlang einer Hohlnadel 28 in das Gewebe eingebracht, wobei die Hohlnadel 28 nach dem Einstechen wieder aus dem Gewebe zurückgezogen wird, und nur der Katheter im Gewebe verbleibt. Die zumindest eine Öffnung 6 ist bevorzugt an einem Ende der Nadel bzw. des Katheters 2 angebracht, wobei an dieser Stelle die Septen an der Oberfläche der Leitungen 7 und 13 fehlen können, sodaß aus allen kapillaren Spalten die Perfusionsflüssigkeit durch zumindest eine Öffnung 6 in die abführende Leitung 7 gelangen kann.

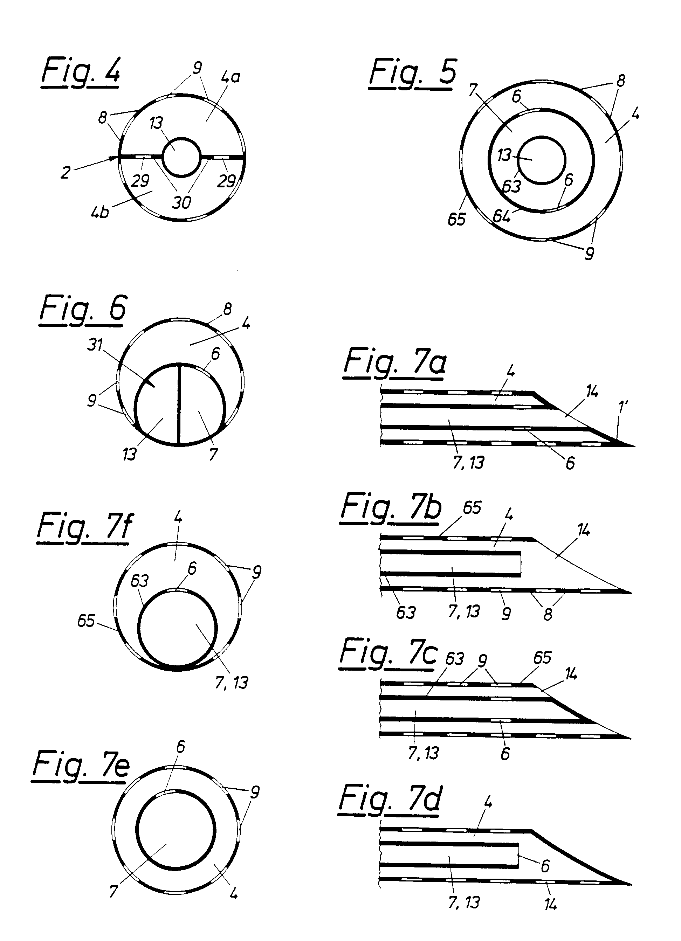

Fig. 4 zeigt eine weitere mögliche Ausführungsform, bei welcher innerhalb der Subcutannadel/-katheter 2 die Zuleitung 13 für das Medikament angebracht ist und der Austauschkanal 4 durch eine Trennwand 30 zweigeteilt ist. Dabei steht der mit 4a bezeichnete Teil des Austauschkanals 4 mit der hier nicht dargestellten zuführenden Leitung 3 in Verbindung. Auf der Höhe des Schnittes II-II in Fig. 1 krümmt sich der Kanal 4a durch die Öffnungen 29 in den zweiten Teil 4b des Kanals, der mit der abführenden Leitung in Verbindung steht. Durch diese Ausführung kann sowohl der eine als auch der andere Weg zur Equilibration verwendet werden, es muß jedoch darauf geachtet werden, einen Shunt der Perfusionsflüssigkeit an der Außenseite der Nadel zu verhindern, weil durch den Shunt nicht die gesamte Länge der Nadel zur Equilibration benützt würde. Diese Ausführung kann auch mit durchgehender Trennwand 30 ohne Medikamentenleitung 13 verwirklicht werden.

In Fig. 5 ist eine Ausführungsform der Subcutannadel/-katheter 2 dargestellt, bei der drei zylindrische Kanülen 63, 64, 65 konzentrisch ineinander angeordnet sind. In der innersten 63 befindet sich die Zuleitung 13 für das Medikament, das am offenen Ende dieser Kanüle in den Körper austritt. In der mittleren Kanüle 64 wird die Perfusionsflüssigkeit transportiert, wobei die äußere Kanüle 65 den Austauschkanal 4 bildet. Die Öffnung 6 zwischen Kanal 4 und Leitung 7 ist vorzugsweise nahe der Spitze der Nadel angebracht und zwar am besten am distalen Ende der Nadel, um hier wieder die gesamte Länge der Subcutannadel als Perfusionsstrecke auszunützen. Die mittlere und äußere Kanüle sind beispielsweise am Ende der Nadel blind verschlossen. Die dargestellte Form ist auch leicht maschinell zu fertigen und könnte sehr dünnwandig ausgeführt sein, wobei zwischen den konzentrischen Kanülen 63, 64, 65 nur ein kapillarer Spalt vorhanden sein müßte.

Fig. 6 zeigt schließlich eine Ausführungsform des Subcutankatheters oder der Subcutannadel 2 bei der das Innere durch ein, z.B. verzweigtes Septum 31 oder zwei Septen in drei Abschnitte zerlegt wird, wobei ein Abschnitt als Austauschkanal 4 ausgebildet und an die Zuleitung 3 angeschlossen ist. Das Septum 31 sollte dabei so gestaltet sein, daß ein möglichst großer Umfang der Nadel als Perfusionsstrecke dienen kann. Obwohl ein Teil der Nadeloberfläche dabei als Equilibrationsfläche verlorengeht, wird dies durch den geringeren Materialverbrauch und damit durch eine dünnere Nadel oder Kanüle, die schmerzfreier einzustechen ist, wettgemacht.

Die Fig. 7a bis 7f zeigen weitere mögliche Ausführungsformen von Subcutannadeln 2, wobei die Fig. 7a bis 7d Längsschnitte entsprechend dem Teil 1 in Fig. 1 und die Fig. 7e und 7f Querschnitte entsprechend Fig. 2 zeigen.

Gerade wenn die Nadel wiederholt in den Körper eingestochen werden soll, kann es äußerst wichtig sein, die Nadel oder den Katheter mit möglichst geringem Durchmesser auszuführen. Für die Ausbildung einer Perfusionsstrecke und einer Zufuhr für die Medikamentenapplikation genügt in einigen Fällen auch eine zweilumige Subcutannadel/-katheter entsprechend Fig. 7a bis 7f, wobei im Bereich der Spitze 1' der Nadel eine Medikamentenöffnung 14 vorhanden sein kann. Bei der Perfusion der Nadel oder des Katheters wird in üblicher Weise die Perfusionsflüssigkeit durch den Austauschkanal 4 geleitet und durch den Sog am Ende der Perfusionsstrecke durch zumindest eine Öffnung 6 in die abführende Leitung 7 gelangen. Die abführende Leitung 7 kann gleichzeitig als Medikamentenzuleitung 13 dienen, wenn man diese intermittierend benützt, um mit Überdruck das Medikament durch die Leitung 7 an die Öffnung 14 zu bringen. Nach der Medikamentenapplikation kann man z.B. die Perfusionsflüssigkeit dazu benützen, um auch die Reste des Medikaments aus der Nadel zu spülen, sodaß der Patient tatsächlich die gesamte errechnete Medikamentenmenge erhält. Während des Perfusionsbetriebes würde die Medikamentenöffnung 14 keinen Nachteil darstellen, da diese Öffnung dann durch den anliegenden Unterdruck in der Leitung 7 ohnehin durch anliegendes Gewebe verschlossen wäre.

Eine besonders einfach herzustellende Ausführungsform einer zweikanaligen Subcutannadel/-katheter wird, z.B. in Fig. 7b gezeigt. Man führt hier in eine äußere Kanüle 65 mit seitlichen Öffnungen 9 eine weitere Kanüle 63 von geringerem Durchmesser ein. Vor dem Einführen der inneren Kanüle 63, welche den abführenden Kanal 7 bildet, kann man an der äußeren 65 leicht die notwendigen Öffnungen 9 anbringen, beispielsweise durch Applizieren eines Energiestrahles, wie z.B. durch einen Laserstrahl. Vorteilhafterweise werden mehrere Reihen von Öffnungen angebracht, von denen jeweils die auf der Gegenseite der Kanüle liegenden Öffnungen 9 deckungsgleich angebracht werden. Besonders vorteilhaft ist es dabei, die innere Kanüle 63 nicht ganz an die Spitze 1' der äußeren Kanüle 65 heranzuführen, sodaß diese nicht durch anliegendes Körpergewebe verschlossen werden kann.

Es sind natürlich auch Ausführungsformen denkbar, bei welchen, wie in Fig. 7c dargestellt, die innere Kanüle 63 verschlossen ausgeführt ist und das Medikament über die Öffnung 6 und die ringförmige Öffnung 14 der äußeren Kanüle 65 im Bereich der Spitze 1' der Nadel ins Gewebe austritt.

Schließlich ist eine Ausführung 7d, ähnlich der nach Fig. 7b möglich, bei welcher jedoch die äußere Kanüle 65 eine die Spitze 1' formende Abdeckung aufweist und das Medikament durch Öffnungen 9 im Bereich der Spitze 1' austritt.

Wie in der Fig. 7e dargestellt, kann man durch Anordnung der abführenden Leitung 7 im Zentrum der Subcutannadel den gesamten Umfang derselben als Equilibrationsstrecke nützen, oder wie in Fig. 7f gezeigt, die Nadel in einen Austauschkanal 4 und eine abführende Leitung 7 teilen, wobei die innere Kanüle 63 an der äußeren 65 anliegt. Die Öffnungen 9 können so über den größten Teil des Umfangs der Subcutannadel angeordnet sein und fehlen nur dort, wo die abführende Leitung 7 an der äußeren Kanüle 65 anliegt.

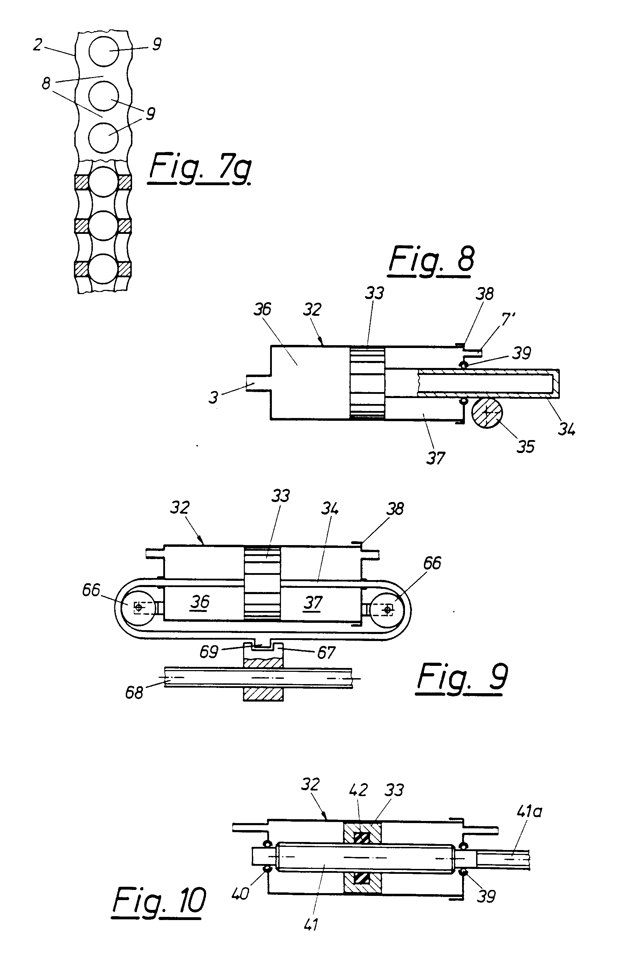

Fig. 7g zeigt die Anordnung der Öffnungen 9 einer Subcutannadel 2 im Detail. Bewährt hat sich z.B. eine Subcutannadel/Kanüle mit einem minimalen Außendurchmesser von ca. 0,5 mm und einer Länge von ca. 20 bis 30 mm. Diese Nadel kann z.B. auf eine Länge von ca. 18 mm mit Öffnungen 9 ausgestattet werden, wobei sich z.B. vier Reihen von Löchern mit ca. 0,12 mm Durchmesser verteilt über die Zirkumferenz anbieten, die, wenn sie in einem Abstand von ca. 0,6 mm (Abstand der Lochmitten) längs der Nadel angebracht sind, ca. 30 Bohrungen in einer Reihe und damit insgesamt ca. 120 Bohrungen mit einer Austauschfläche von mindestens $0,0113 \times 120 = 1,36$ mm$^2$ ergeben würde. Im Inneren der Subcutannadel, die beispielsweise einen Innendurchmesser von 0,3 mm aufweisen kann, kann eine weitere Hohlkanüle mit ca. 0,2 mm Außendurchmesser und ca. 0,1 mm Innendurchmesser ausgeführt sein. Das Füllvolumen der Nadel/Kanüle in der Perfusionsstrecke beträgt dann nur weniger als 1 $\mu$l. Der Herstellungsvorgang könnte dabei so sein, daß die zwei gegenüberliegenden Bohrungen in einem Arbeitsgang erzeugt werden und die um 90° an der Zirkumferenz versetzten Bohrungen in einem zweiten Arbeitsgang erzeugt werden. Um genügend Material an der Nadelwand zu belassen, um eine geügende Steifigkeit und Bruchsicherheit zu garantieren, sollten die um 90° versetzt an der Zirkumferenz angebrachten Öffnungen z.B. auch in der Längsachse versetzt werden, wie dies in der Abbildung dargestellt ist. Es ist natürlich auch möglich, eine oder mehrere Reihen von Öffnungen 9 spiralig anzuordnen.

In einer weiteren Version kann die Austauschfläche durch Öffnungen gestaltet werden, die nur ca. 80$\mu$m Durchmesser aufweisen, und die damit deutlicher kleiner sind als der Innendurchmesser der abführenden Kanüle. Dies hat den Vorteil, daß Gewebsmaterial, welches durch die Öffnungen eventuell in die Nadel eindringt, nicht die abführende Kanüle verlegen kann. Weiters könnte man die abführende Leitung 7 mit mehreren Öffnungen am Ende derselben ausführen, um so noch sicherer eine Verstopfung zu vermeiden.

Wie aus Fig. 1 ersichtlich, weist die Vorrichtung zahlreiche Behälter für die Perfusionsflüssigkeit, für die Kalibrierlösung und das Medikament bzw. zum Auffangen der verwendeten Flüssigkeiten auf, sowie zahlreiche Druck- und Saugpumpen. Um trotzdem eine kleine, tragbare Ausführungsform zu gewährleisten und gleichzeitig den Aufbau der Pumpen zu vereinfachen, wird in Fig. 8 eine mögliche Ausführung einer Pumpe dargestellt, welche gleichzeitig als Druckpumpe 15 und Saugpumpe 16 betrieben werden kann. Dabei ist mit 32 eine Kolbenpumpe gekennzeichnet, deren Kolben 33 bei der Vorwärtsbewegung des Antriebsstempels 34, z.B. mittels eines Reibrades 35, den Raum 36 vor dem Kolben verkleinert, dabei jedoch gleichzeitig im Kolbenraum 37 ein Vakuum schafft, da das Ende der Kolbenpumpe 32 mit einem Deckel 38 versehen ist, sodaß der Raum 37 gleichzeitig als Auffangbehälter 18 verwendet werden kann. Dafür ist es notwendig, daß der Deckel 38 gegen den Stempel 34, z.B. mit einem O-Ring 39 abgedichtet wird. Dadurch, daß der freiwerdende Raum 37 als Auffangbehälter genützt wird, ist einerseits eine Verkleinerung der Anordnung gegeben und andererseits nur eine Pumpe notwendig, um sowohl Pump- als auch Saugfunktionen zu bewerkstelligen. Die unterschiedlichen Pump- und Saugvolumina dieser Pumpe, die durch die Anwesenheit des Antriebsstempels 34 nur im Raum 37 entstehen, müßten nicht unbedingt ein Nachteil sein, wenn unterschiedlich große Volumina für Reservoire und Auffangbehälter benötigt werden.

Um die Außenmaße der Pump- und Saugvorrichtung weiter zu verkleinern, kann man, wie in Fig. 9 dargestellt, den Antriebsstempel 34 auch biegsam ausführen, ihn entlang der Kolbenpumpe 32 mittels Umlenkteilen oder Rollen 66 umlenken durch einen weiteren O-Ring abdichten und durch den Raum 36 wieder an den Kolben 33 heranführen. Mit einem einzelnen Antrieb, beispielsweise einem Reibrad 35 wie in Fig. 8, hat man so eine äußerst platzsparende Druck- und Saugvorrichtung verwirklicht. Die Ausführungsform mit dem Antriebsstempel 34 auf beiden Seiten des Kolbens 33 hat den Vorteil, daß die Pump- und Saugvolumina identisch sind. Der biegsame Stempel 34 kann auch als Seil oder als mit dem Kolben 33 einstük-

kig hergestellter Kunststoffteil ausgeführt sein. Der Antrieb des Stempels 34 kann durch eine Mutter 67, die entlang einer Spindel oder Schraube 68 bewegt wird, erfolgen. Der Stempel 34 kann dabei durch eine Änderung der Form, z.B. durch eine Nase 69, beim Auswechseln der Druck- und Saugvorrichtung an der Mutter 67 einrasten, sodaß der komplette Antrieb, bestehend aus Mutter 67 und Schraube bzw. Spindel 68, in der Vorrichtung verbleiben kann. Wenn der Stempel 34 nicht nur biegsam ausgeführt ist, sondern auch aus komprimierbarem Material besteht, kann man sich die zur Abdichtung des Antriebsstempels 34 gegenüber dem Gehäusedeckel 38 notwendigen O-Ringe auch ersparen. So könnte z.B. der Antriebsstempel 34 aus zwei Materialien, z.B. aus einem zugfesten und biegsamen Kern, wie z.B. Teflon und einem komprimierbaren Material wie z.B. Silicon, gefertigt sein.

Fig. 10 zeigt eine weitere mögliche Ausführungsform einer Kolbenpumpe, bei der der Antriebsstempel 34 als Gewinde 41 ausgeführt ist, und am äußeren freien Ende 41a, beispielsweise von einem Reibrad in Drehbewegung, versetzt wird. Dadurch, daß der Kolben 33 durch seine Formgebung (z.B.vier- oder mehreckig oder oval statt kreisrund) oder durch die Reibung an den Wand der Kolbenpumpe 32 am Mitdrehen gehindert wird, wird die Drehbewegung des Stempels 34 in eine Längsbewegung des Kolbens 33 umgesetzt. Diese Vorrichtung, die äußerst wenig Platz beansprucht, ist auch leicht herzustellen, wenn das Gewinde an beiden Seiten, wie in der Fig. 10 dargestellt, dort wo der Stempel 34 die Kolbenpumpe 32 verläßt, glatte Stellen aufweist, sodaß der Stempel 34 hier, beispielweise durch O-Ringe 39 und 40, gegen die Wand der Kolbenpumpe 32 abzudichten ist. Der Kolben 33 muß gegebenenfalls auch durch eine zusätzliche Dichtung 42 gegenüber dem Stempel abgedichtet werden. Wenn jedoch der Kolben 33 aus entsprechend elastischem Material hergestellt ist, wird sich in der Regel eine zusätzliche Dichtung vermeiden lassen.

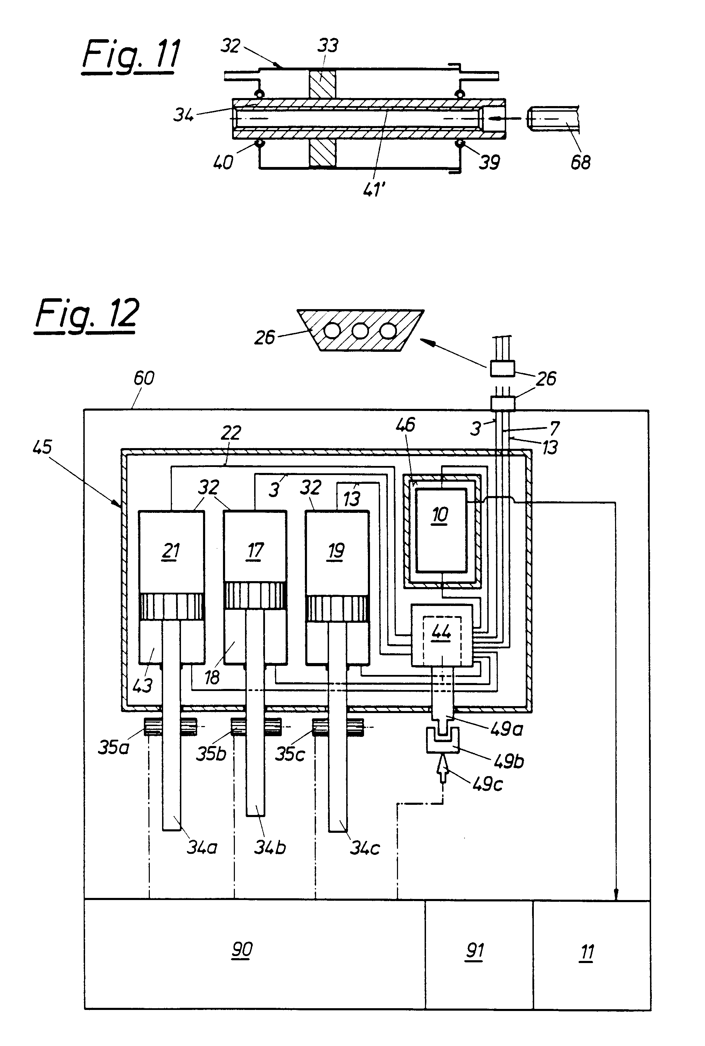

Fig. 11 zeigt eine weitere Ausführungsform in Abwandlung der Fig. 10. Der Antriebsstempel 34 ist hier hohl ausgeführt und weist an seiner Innenseite ein Gewinde 41' auf, das z. B. von einer Schraube 68, je nach Drehrichtung entweder eine Vorwärts- oder Rückwärtsbewegung mitgeteilt bekommt, und so den Kolben 33 vorwärts oder rückwärts bewegt. Durch die Formgebung des Kolbens 33 im Gehäuse wird wieder ein Mitdrehen vom Stempel 34 und Kolben 33 verhindert. Das gepumpte und gesaugte Volumen ist auch bei dieser Ausführungsform identisch.

Um die Handhabung des Gerätes durch den Patienten zu vereinfachen, ist es möglich, alle auszuwechselnden Teile in ein komplettes Einmal-Set zu vereinen, welches ausgetauscht werden kann. Fig. 12 zeigt eine derart gestaltete auswechselbare Einheit. Das Reservoir 19 für das Medikament das Reservoir 17 für die Perfusionsflüssigkeit, der Auffangbehälter 18 für die verwendete Perfusionsflüssigkeit, der Behälter 21 für die Kalibrierlösung, der Auffangbehälter 43 für die Kalibrierlösung sowie ein noch näher zu beschreibendes Mehrwegventil 44 sind dabei auf einem gemeinsamen Träger oder in einem gemeinsamen Gehäuse 45 untergebracht, das nicht nur alle verwendeten Lösungen und Flüssigkeiten im System gegen die Elektronik der Vorrichtung abdichtet, sondern auch eine problemlose Handhabung durch den Patienten gewährleistet. So braucht dieser nur den gesamten Träger 45 gegen einen neuen austauschen und in das Gehäuse 60 einsetzen. Über die gemeinsame Steckverbindung 26 werden die Leitungen 3, 7 und 13 an die hier nicht dargestllte Subcutannadel angeschlossen. Die drei Antriebsstempel 34a bis 34c der Kolbenpumpen 32 werden bei Einlegen des Trägers 45 in das Gehäuse 60 der Vorrichtung automatisch mit dem Antrieb im gezeichneten Fall mit den Reibrädern 35a bis 35c in Kontakt gebracht. Die Analyseneinrichtung 10 kann sich entweder ebenfalls auf dem Träger 45 befinden und mit diesem ausgewechselt werden oder auch in einer Aussparung 46 des Trägers 45 vorliegen, um so separat ausgewechselt werden zu können. Die hier nur schematisch gezeichnete Antriebseinheit 90 weist eine Energieversorgung 91 auf, und treibt in schematisch strichpunktiert dargestellter Weise die Reibräder 35a bis 35c an. Es ist natürlich auch möglich, für den Antrieb der einzelnen Pumpen bzw. der Reibräder 35a bis 35c separate Motoren zu verwenden. Mit 49a bis c ist der Antrieb des Mehrwegventils bezeichnet.

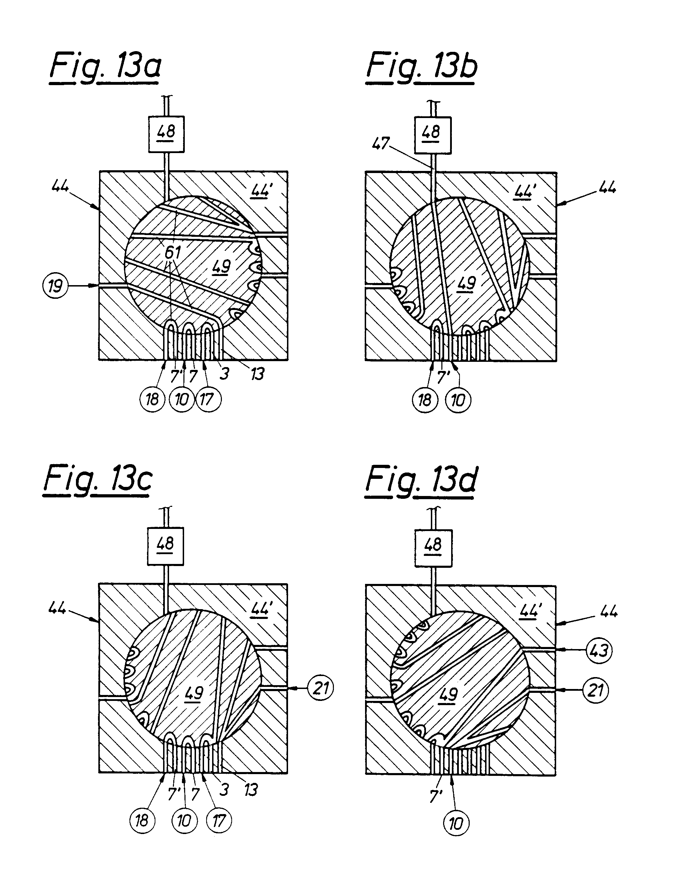

Fig. 13 zeigt eine beispielsweise Detailansicht eines Mehrwegventils 44, welches durch mehrere unabhängige, im als Drehkörper ausgeführten beweglichen Teil 49 angeordnete Kanäle 61 folgende Verbindungen herstellen kann. Die im folgenden angeführten zugeschalteten Behälter und Bauteile werden durch ihr in ein Kreiszeichen gesetztes Bezugszeichen verdeutlicht:

In der in Fig. 13a gezeigten Stellung sind gleichzeitig Verbindungen zwischen dem Reservoir 17 für die Perfusionsflüssigkeit und der zuführenden Leitung 3, zwischen der abführenden Leitung 7 und der Analyseneinrichtung 10, zwischen der von der Analyseneinrichtung 10 wegführenden Leitung 7' und dem Auffangbehälter 18, sowie zwischen dem Reservoir 19 für das Medikament und der Medimentenleitung 13 hergestellt.

In Fig. 13b sind Verbindungen zwischen einer mit der Außenluft in Verbindung stehenden Öffnung 47 im Ventilkörper 44' und der Analyseneinrichtung 10, sowie zwischen der Leitung 7' und dem Auffangbehälter 18 hergestellt.

In der in Fig. 13c dargestellten Stellung sind gleichzeitig Verbindungen zwischen Perfusatreservoir 17 und zuführender Leitung 3, zwischen abführender Leitung 7 und Analyseneinrichtung 10, zwischen Leitung 7' und Auffangbehälter 18, sowie zwischen dem Behälter 21 für die Kalibrierlösung und der Medikamentenleitung 13 hergestellt.

In der Stellung nach Fig. 13d schließlich sind gleichzeitig Verbindungen zwischen dem Behälter 21 für die Kalibrierlösung und der Analyseneinrichtung 10, zwischen Leitung 7' und dem Auffangbehälter 43 für die Kalibrierlösung hergestellt.

Die beschriebene oder eine ähnliche Ausführungsform hat den Vorteil, daß mit einem einzigen Mehrwegventil und damit mit einer einzigen Drehbewegung, sowohl wahlweise die Perfusionsflüssigkeit von der Analyseneinrichtung 10 gemessen, als auch die Sensoren der Analyseneinrichtung 10 durch die Kalibrierlösung nachgeeicht werden können.

Die in Fig. 13a gezeigte Stellung dient zur Perfusion der Subcutannadel, wobei die wiedergewonnene Perfusionsflüssigkeit laufend von der Analyseneinheit 10 analysiert wird und der Rechner der Auswerteeinheit 11 über die zwischen Medikamentenbehälter 19 und Nadel hergestellte Verbindung laufend die notwendige Medikamentenmenge appliziert.

Die Stellung nach Fig. 13b dient dazu, zwischen dem Kalibriervorgang und der Analyse eine Luftblase durch die Öffnung 47 anzusaugen, um zu verhindern, daß sich die zu messende Perfusionsflüssigkeit und die Kalibrierlösung miteinander vermischen. Gleichzeitig läßt sich durch das Mehrwegventil auch ein allfälliges Ventil 25a (Fig. 1) ersetzen, welches die Analyseneinrichtung 10 gegen den Auffangbehälter 18 oder 43 abschließt oder freigibt.

Die in Fig. 13c gezeigte Ventilstellung dient dazu, einerseits den Austauschkanal über die zuführende Leitung 3 mit Perfusionsflüssigkeit aus dem Behälter 17 zu versorgen, und diese nach Equilibration durch die abführende Leitung 7 an die Analyseneinrichtung 10 heranzubringen, andererseits ist hier statt des Medikamentes aus dem Behälter 19 der Behälter 21 mit der Kalibrierlösung an die Medikamentenleitung 13 angeschlossen. Sollten nämlich bei Verwendung der Vorrichtung als künstliche Bauchspeicheldrüse einmal die Zuckerwerte zu stark abfallen, müßte eventuell die Insulinzufuhr durch die Leitung 13 für längere Zeit unterbrochen werden, wodurch sich die Medikamentenöffnung in der Nadel verschließen könnte. Um dies zu verhindern, müßte man die Leitung 13 durch eine andere Flüssigkeit offenhalten, wie dies in Fig. 13c gezeigt ist. Da bei einem zu niedrigen Blutzucker auch die Verabreichung einer blutzuckkersteigernden Substanz von Vorteil wäre, könnte man dieser Flüssigkeit, die zum Offenhalten der Leitung 13 und der Medikamentenöffnung 14 dient, auch eine blutzuckersteigernde Substanz, z.B. Glucagon zusetzen. In gewissen Fällen könnte sich als Flüssigkeit für das Offenhalten der Medikamentenleitung 13 auch die Perfusionsflüssigkeit eignen, speziell dann, wenn die Sensoren und die Elektroden der Analyseneinrichtung 10 so stabil sind, daß sie nicht nachgeeicht werden müssen. In diesem Fall könnte der zusätzliche Behälter für die Kalibrierlösung entfallen.

Bei der Kalibrierung nach der Fig. 13d wird die Kalibrierflüssigkeit vom Behälter 21 in die Analyseneinrichtung 10 gebracht und gelangt in der Folge über die Leitung 7' in den Auffangbehälter 43. Eine weitere wichtige, hier nicht dargestellte Ventilstellung stellt die Verbindung zwischen dem Behälter für die Perfusionsflüssigkeit und der Analyseneinrichtung unter Umgehung des Austauschkanales dar, um so mit einer ionen- und glucosearmen Perfusionsflüssigkeit eine Nullpunktskalibrierung vorzunehmen.

Da die beispielsweise in der in Fig. 13b dargerstellten Position angesaugte Luft mit den Flüssigkeiten, die Kontakt zum lebenden Organismus haben, in Berührung kommt, kann man an der Eintrittsstelle für die Luft ein Bakterienfilter 48, beispielsweise in der Öffnung 47, anbringen. Der Verlauf der Kanäle 61 im beweglichen Teil 49 des Mehrwegventils 44 in den Fig. 13a bis 13b ist rein schematisch dargestellt und soll nur die Praktikabilität eines solchen Mehrwegventils veranschaulichen. In der Praxis wird man die Kanäle 61 in mehreren Ebenen anordnen und so einen kreuzungsfreien, geradlinigen Verlauf erreichen. In einer anderen Ausgestaltung wäre es auch möglich, anstatt der Kanäle 61 an der Oberfläche des drehbaren Teils 49 geführte Nuten vorzusehen, welche die notwendigen Verbindungen schaffen.

Es soll auch noch betont werden, daß das Mehrwegventil 44 noch viele andere wichtige Funktionen übernehmen kann, wie z.B. Umkehrung der Strömungsrichtung der Perfusionsflüssigkeit in der Subcutannadel u.s.w.

In jeder beliebigen Zwischenstellung des Mehrwegventils 44 ist gleichzeitig die Analyseneinrichtung 10 von der abführenden Leitung 7 bzw. vom Behälter 21 mit der Kalibrierlösung sowie von der Leitung 7' zu den Auffangbehältern 18 und 43 getrennt, sodaß die Analyseneinrichtung ausgetauscht werden kann, ohne die Druckverhältnisse in den Leitungen oder den Behältern zu verändern. Dies hat den Vorteil, daß ein einmal für den Transport der Flüssigkeit aufgebauter Unter- oder Überdruck nicht mehr verloren gehen kann. Wenn nämlich der Druck erst wieder aufgebaut werden müßte, würde Platz in den einzelnen Behältern verloren gehen. Die in Fig. 13a bis 13d gezeigten Stellungen des Mehrwegventils 44 könnten auch dazu benützt werden, z.B. ein Getriebe so zu steuern, daß jede Ventilstellung den Antrieb der zu diesen Betriebszustand gehörenden Pumpen bewirkt. So wäre z.B. bei der in Fig. 13a gezeigten Stellung der Antrieb 35b für die Perfusatpumpe sowie eventuell für die Medikamentenpumpe eingeschaltet, bei der in Fig. 13b gezeigten Ventilstellung wäre der Antrieb 35a für die Perfusatpumpe eingeschaltet. Und bei der in Fig. 13c gezeigten Stellung wäre die Perfusatpumpe sowie die Pumpe für die Kalibrierlösung eingekuppelt. Bei der in Fig. 13d gezeigten Ventilstellung könnte, z.B. die Kalibrierpumpe, allein eingekuppelt sein. Die Vorrichtung kann dadurch mit einer einzigen Antriebseinheit 90 und deren Energieversorgung 91 (siehe Fig. 12) betrieben werden, welche beispielsweise über Zahnstangen oder über ein nicht weiter dargestelltes Hebelwerk alle mechanischen Funktionen ausführt. Die Kopplung des Trägers 45, der alle Behälter, Pumpen, Leitungen und das Mehrwegventil enthält, mit der Antriebseinheit 90 ist, wie in Fig. 12 schematisch dargestellt, sehr einfach, da durch Plazieren des Trägers in der Vorrichtung nicht nur die als Reibräder ausgeführten Antriebe für die Stempel der Pumpen gewährleistet sind, sondern gleichzeitig auch eine Kupplung zum Mehrwegventil 44 hergestellt werden kann. Dieses kann über eine Nabe 49a, in welcher der Antrieb 49b eingreift, mit der Antriebseinheit 90 Kontakt aufnehmen. Damit unabhängig von der Stellung des Mehrwegventils 44 immer der Antrieb 49b richtig mit dem Mehrwegventil verbunden wird, ist die Nabe 49a und deren Gegenstück, der Antrieb 49b, asymmetrisch gestaltet und zusätzlich mit einer Feder 49c ausgestattet, sodaß erst bei richtiger Stellung der beiden Teile zueinander die Nabe 49a in den Antrieb 49b einrasten kann.

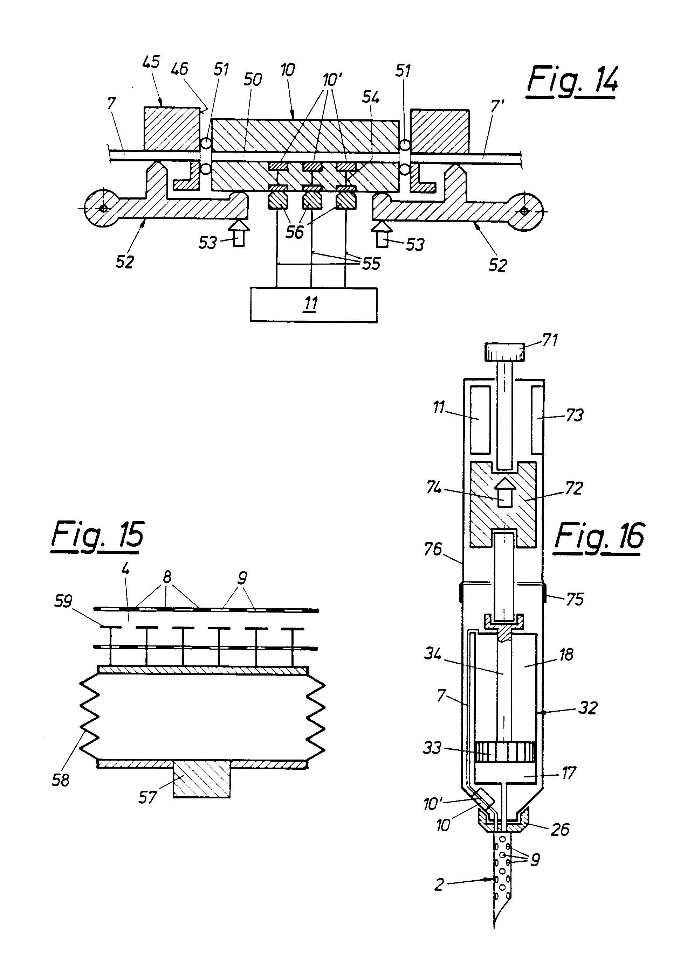

Fig. 14 zeigt die Analyseneinrichtung 10 als Block, welcher im Fall eines Defektes separat ausgetauscht werden kann. Im eingesetzten Zustand ist die Meßkapillare 50 der Analyseneinheit 10 mittels O-Ringen 51 gegen die Leitungen 7 und 7' abgedichtet. Um bei Entfernung der Analyseneinheit 10 eine Abdichtung der abführenden Leitung 7 und der zum Auffangbehälter führenden Leitung 7' zu bewirken, und so einen Druckverlust im System zu verhindern, ist z.B. ein Verschließmechanismus 52 vorgesehen, welcher über Federn 53 ein Abklemmen der Leitungen 7, 7' bewirkt. Beim Einsetzen der Analyseneinrichtung 10 in die Aussparung 46 des Trägers oder Gehäuses 45 werden die elektrischen Leitungen 54 der einzelnen Sensoren und Meßelektroden 10' der Analyseneinrichtung 10 automatisch mit den Zuleitungen 55 zur Auswerteeinheit 11 über elektrische Kontakte 56 verbunden.

Die gesamte Vorrichtung läßt sich auch implantierbar gestalten, indem man an der Oberfläche des implantierten Gerätes einen Austauschkanal 4 vorsieht und große Teile der Oberfläche als Equilibrationsfläche benützt. Die Reservoire 17 bzw. 19 für die Perfusionsflüssigkeit bzw. das Medikament könnten bei einer implantierbaren Vorrichtung, wie in Fig. 15 dargestellt, über ein Septum 57 transcutan mittels einer Nadel nachgefüllt werden. Durch die Vergrößerung und Verkleinerung beim Nachfüllen und Entleeren, beispielsweise des Reservoirs 17, welches über verformbare Wandteile 58 verfügt, kann eine mechanische Einrichtung 59, z.B. Stempel, die an der Wand des Reservoirs befestigt sind, angetrieben werden, durch welche der Austauschkanal 4 an der Oberfläche des Gerätes mechanisch von eventuell einsprießendem Gewebe freigehalten werden kann. Durch die Haut müßte man so nur von Zeit zu Zeit das Reservoir für die Perfusionsflüssigkeit bzw. das Medikamentenreservoir nachfüllen. Ein Auffangraum für die verbrauchte Perfusionsflüssigkeit kann hier eventuell entfallen, weil die kleinen Flüssigkeitsmengen nach dem Messen vom Gewebe resorbiert werden können. Es muß betont werden, daß es sich bei den beschriebenen Ausführungsformen nur um Beispiele handelt, und daß viele weitere Ausführungsformen vorstellbar sind.

Bekanntlich gibt es erfolgreiche Bestrebungen, mit Hilfe von handbetriebenen Pumpen Medikamente in den Körper zu injizieren. So sind z.B. Vorrichtungen verwirklicht, bei denen eine Medikamentenampulle in einem schreibstiftähnlichen Gehäuse untergebracht ist, wobei durch Druck eines Hebels oder Drehung eines Rades der Kolben der Ampulle um das gewünschte Maß nach vorne verschoben werden kann, um eine genau definierte Medikamentenmenge in den Körper zu bringen.

Fig. 16 zeigt eine beispielsweise Ausführungsform eines "Glucose-Pen", bei welchem mit 70 ein annähernd schreibstiftförmig gestaltetes Gehäuse zu sehen ist, das an seinem einen Ende eine Subcutannadel 2 aufweist. Im Inneren des Gehäuses 70 befinden sich z.B. eine Kolbenpumpe 32 mit einem Reservoir 17 für die Perfusionsflüssigkeit, aus welchem mit Hilfe des Kolbens 33 sowohl die Perfusionsflüssigkeit herausgepumpt als auch in den Auffangbehälter 18 angesaugt werden kann. So kann z.B. mit Hilfe einer Hebeleinrichtung 71 über ein allenfalls notwendiges, schematisch gezeichnetes Getriebe 72 der Kolben manuell bewegt werden und Perfusionsflüssigkeit in der beschriebenen Weise an die Öffnungen 9 der Subcutannadel 2 gebracht und gleichzeitig wieder durch die abführende Leitung 7 durch den Sog im Auffangbehälter 18 gesammelt werden. Möglichst knapp an der Subcutannadel ist dabei die Analyseneinrichtung 10 mit der Meßkapillare 10' für die zu bestimmende Substanz und die Markersubstanz bzw. die Markergröße angebracht. Es kann somit für kurze Zeit, z.B. in der Größenordnung von ca. 1 Minute oder weniger, Flüssigkeit durch die Subcutannadel 2 gepumpt und nach deren Equilibration über die Öffnungen 9 gesammelt werden. Bereits nach kurzer Zeit kann z.B. über eine optische Anzeige 73 oder auch eine akustische Anzeige die wahre, im Gewebe herrschende Konzentration der interessierenden Substanz, z.B. des Blutzuckers, erfaßt werden, die von der Auswerteeinheit 11 aus der Markergröße z.B. der Konzentration der Markersubstanz und der Konzentration der interessierenden Substanz errechnet wird. Um während der erforderlichen Zeit der Gewebeperfusion eine gleichmäßige Flußrate zu erhalten, sind mehrere Vorgangsweisen denkbar. So kann z.B. über die Hebeleinrichtung 71, deren Hebel während der Perfusionsperiode immer wieder betätigt wird, eine gleichmäßige Perfusionsrate erzielt werden. Der Hebel könnte dazu z.B. über eine schematisch gezeichnete Feder 74 oder einen anderen Energiespeicher immer wieder in seine Ausgangslage gebracht werden. Die Perfusionsrate während der Sammelperiode könnte z.B. in der Größenordnung zwischen 1 und 10 $\mu$l/min liegen.

Eine andere, wahrscheinlich attraktivere Möglichkeit ist die, mit einer oder mehreren Betätigungen der Hebeleinrichtung 71 einen Energiespeicher zu laden, welcher dann gleichmäßig während der nächsten Zeit das Getriebe 72 betätigt, wodurch der Flüssigkeitstransport bewerkstelligt wird. Genauso wäre es natürlich auch vorstellbar, in die beschriebene Vorrichtung eine andere Kraftquelle, wie z.B. einen Motor, unterzubringen. Um möglichst sofort ein Meßresultat zu erhalten, wobei die Subcutannadel angenehmerweise nur kurz im Gewebe verbleiben müßte, wäre es günstig, die Strecke zwischen Subcutannadel 2 und Analyseneinrichtung 10 möglichst kurz zu halten, indem wie dargestellt, die Meßkapillare 10' möglichst knapp am Verbindungsstück 26 der Subcutannadel/-katheter 2 liegt. Das Fassungsvolumen der Meßkapillare 10' sollte günstigerweise nur einige $\mu$l oder weniger betragen, um augenblicklich eine Analyse zu ermöglichen. Die Sensoren bzw. die Meßelektrode für die Markersubstanz bzw. Markergröße könnten dabei durch z.B. eine optische, oder akkustische Anzeige eine adequate Füllung der Meßkapillare 10' und/oder adequate Equilibration signalisieren, sodaß der Pumpvorgang dann abgebrochen werden kann.

Das komplette Set mit Reservoir 17 und Auffangbehälter 18 für die Perfusionsflüssigkeit und ggf. die Analyseneinrichtung 10 sollte am besten als Einweg-Set ausgeführt sein, welches nach dem Aufschrauben des Gewindes 75 des Gehäuses 70 aus dem "Glucose-Pen" entfernt und durch ein neues Set ersetzt werden kann. Nach der Messung kann z.B. eine notwendige Insulinapplikation mit Hilfe eines konventionellen Medikamentenapplikators genau dem zuletzt gemessenen Blutzucker angepaßt werden.

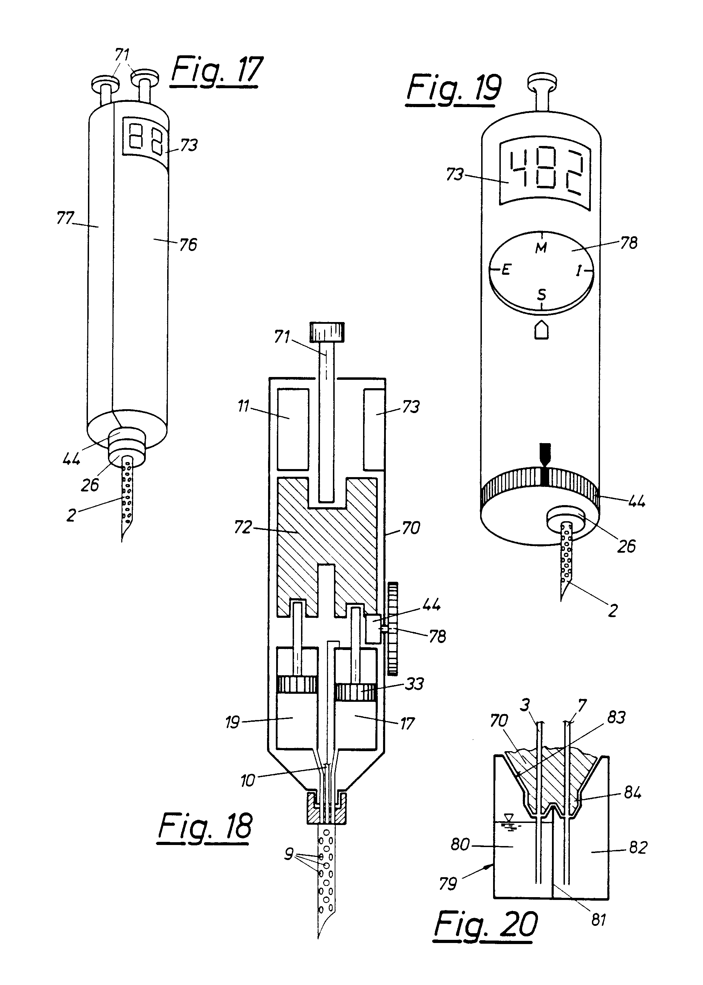

Es wäre auch vorstellbar, z.B. einen "Glucose-Pen" huckepack an einen "Insulin-Pen" zu befestigen, wie das in Fig. 17 gezeigt ist. Beide Vorrichtungen, der Glucose-Pen 76 und der Insulin-Pen 77 wären dabei an dieselbe Subcutannadel 2 über die Steckverbindung 26 angeschlossen, wobei direkt an die Steckverbindung 26 ein Mehrweghahn 44 angeschlossen werden könnte, um die Subcutannadel 2 abwechselnd dem Glucose-Pen 76 und dem Insulin-Pen 77 zuzuschalten. Man kann damit dieselbe Nadel verwenden, um die Störung im Organismus zu messen und dann mit Hilfe einer Medikamenteninjektion zu beheben.

Fig. 18 zeigt eine Ausführungsform, bei der der Glucose-Pen und der Insulin-Pen nicht aneinander befestigt sind sondern in eiem gemeinsamen Gehäuse 70 untergebracht sind. Das Einmal-Set, bestehend aus Medikamentenbehälter 19, Reservoir 17 für die Perfusionsflüssigkeit und ggf. die Analyseneinrichtung 10 kann auch hier als Einheit ausgetauscht werden.

Es ist natürlich auch möglich, daß sich direkt anschließend an die Steckverbindung 26 außerhalb des Gehäuses 70 eine separat auswechselbare Analyseneinrichtung befindet, sodaß die Vorrichtung im wesentlichen aus vier leicht trennbaren Teilen aufgebaut ist, die entsprechend der Häufigkeit des Auswechselns hintereinander angeordnet sind: Der oberste Teil bildet die verbleibende Mechanik mit der zusammengefaßten Auswerte- und Anzeigeneinheit, darunter befestigt sind die zu einem Einmal-Set zusammengefaßten Behälter und Pumpen, welche bei entsprechender Gestaltung einen Teil des Gehäuses bilden können, darunter befindet sich die aufsteckbare Analyseneinrichtung und an diese schließt die am häufigsten gewechselte Subcutannadel an.

In Fig. 19, die eine Außenansicht einer geringfügig geänderten Ausführungsvariante der Vorrichtung zeigt, ist mit 78 ein Stellrad gekennzeichnet, mit Hilfe dessen das Getriebe 72 wahlweise auf den Kolben 33 des Reservoirs 17 für die Perfusionsflüssigkeit oder auf den Kolben des Medikamentenreservoirs 19 umgeschaltet werden kann. Gleichzeitig kann damit auch ein eventuell vorhandenes Mehrwegventil 44 mitbedient werden. Mit Hilfe des Stellrades 78 kann man z.B. das Getriebe von M = Messen auf Medikamentenapplikation (z.B. I für Insulin) oder z.B. auch auf Spülen der Subcutannadel (S = Spülen) nach der Applikation des Medikamentes, bzw. auf E = Eichen bzw. Kalibrieren umschalten. Falls ein Mehrwegventil verwendet wird, ist es günstig darauf zu achten, daß dieses gemeinsam mit den Reservoiren und Auffangbehäl-

15

tern austauschbar ist.

Statt einer einzigen Hebeleinrichtung 71 könnten auch zwei vorhanden sein, wie in Fig. 17 dargestellt, mit denen die Kolben der beiden Reservoire 17 und 19 separat bewegt werden können. Statt der Kolbenpumpen können alle anderen Arten von Pumpen Verwendung finden. Auf der Anzeige 73 am Gehäuse 70 wird der von der Auswerteeinheit 11 errechnete Meßwert angezeigt. Es muß noch betont werden, daß bei der Berechnung und Anzeige der endgültigen Konzentration der interessierenden Substanz auch ggf. die Diffusionskonstanten, der Markersubstanz und der interessierenden Substanz in die Berechnung eingehen sollte, welche von einem Mikroprozessor in der Auswerteeinheit berücksichtigt werden könnten.

Es ist auch möglich, ein Mehrwegventil 44 am vorderen Ende des schreibstiftähnlichen Gehäuses 70 zwischen der Subcutannadel 2 bzw. der Steckverbindung 26 und dem Gehäuse 70 anzubringen, wodurch eine in der Konstruktion besonders einfache Umschaltmöglichkeit des Flüssigkeitsstromes gewährleistet ist. Ist jedoch eine Subcutannadel mit drei Leitungen (siehe Fig. 2 bis 6) vorhanden, erübrigt sich eine Umschaltung des Flüssigkeitsstromes an dieser Stelle, da sowohl der Perfusionsweg als auch die Medikamentenzufuhr unabhängig voneinander sind.

Da die Vorrichtung in den in Fig. 16 bis 19 beschriebenen Ausführungsform jeweils nur kurz über die Subcutannadel mit dem Körper in Verbindung steht und zwischendurch einer Überprüfung zugängig ist, kann leicht für eine Kalibrierung der Analyseneinrichtung gesorgt werden. Wie in Fig. 20 gezeigt wird, kann man die Vorrichtung nach Fig. 16 bis 19 in einen Kalibrierbehälter 79 einführen, welcher mit Kalibrierflüssigkeit gefüllt ist, und die zur Kalibrierung notwendigen Komponenten in gewünschter Konzentration enthält. Der Kalibrierbehälter 79 weist eine Öffnung 83 auf, welche in ihrer Form der Steckverbindung 26 entspricht, sodaß der Nadelansatz 84 des Gehäuses 70 luftdicht an den Behälter 79 angeschlossen werden kann. Über die Pumpvorrichtung des Glucose- bzw. Glucose-Insulin-Pen wird dann Kalibrierlösung an die Analyseneinrichtung 10 herangebracht. Der Kalibrierbehälter 79 ist durch eine Trennwand 81 in Teilbehälter 80, 82 geteilt, wodurch gewährleistet ist, daß sich die aus der Vorrichtung austretende Perfusionsflüssigkeit nicht mit der Kalibrierlösung im Teilbehälter 80 vermischen kann. Diese Trennwand 81 könnte auch verformbar sein, um kein Problem mit einem allfälligen Über- oder Unterdruck in einem der Teilbehälter 80, 82 aufkommen zu lassen. Eine asymmetrisch ausgebildete Öffnung 83 des Kalibrierbehälters 79 sorgt dafür, daß das Kalibriergefäß nicht falsch angeschlossen werden kann.

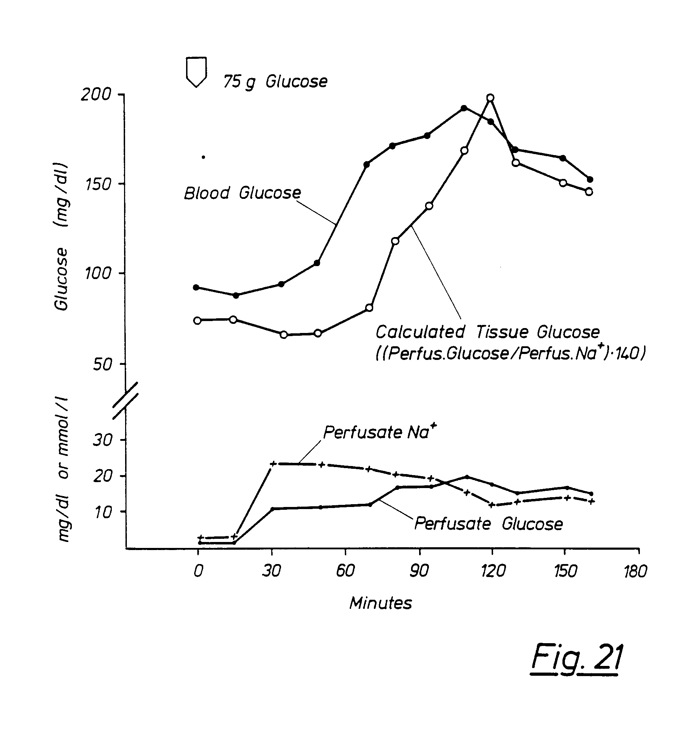

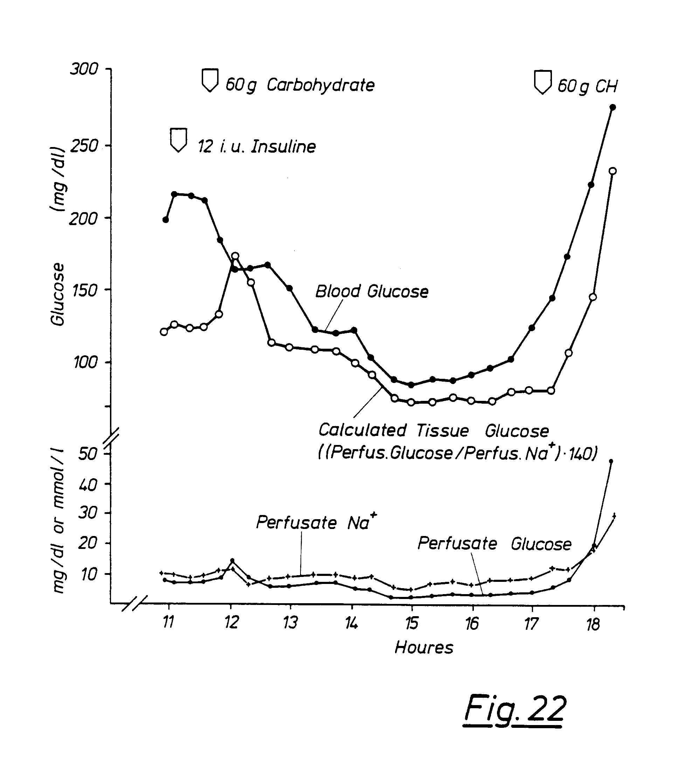

In den in den Fig. 21 und 22 dargestellten Diagrammen ist auf der Abszisse die Zeit in Stunden bzw. Minuten und auf der Ordinate die Konzentration von Glucose bzw. Na$^+$ in mg/dl bzw. mmol/l aufgetragen.

Die Fig. 21 zeigt eine mit Hilfe der erfindungsgemäßen Vorrichtung ermittelte Gewebszuckerkurve im Vergleich mit einer nach herkömmlichen Verfahren gewonnenen Blutzuckerkurve. Wie im unteren Teil der Abbildung ersichtlich, ergibt sich in der wiedergewonnenen Perfusionsflüssigkeit eine Natriumionenkonzentration zwischen 2 und 25 mmol/l. Abhängig von wechselnden physikalischen Faktoren schwankt die Natriumionenkonzentration stark, was auf einen ungleichmäßigen Equilibrierungsgrad schließen läßt. Eine Analyse ausschließlich auf die Glucosekonzentration in der Perfusionsflüssigkeit ist aus diesem Grund äußerst unzureichend. Erst durch die Erfassung einer Markergröße, in diesem Falle der Natriumionenkonzentration, können die Schwankungen im Equilibrationsgrad ausgeglichen werden. Wie ersichtlich, liegt die Glucosekonzentration in der Perfusionsflüssigkeit zwischen 2 und 30 mg/dl, sie liegt damit in einem für die Enzymelektrode optimalem Bereich.

Wie im oberen Teil der Abbildung ersichtlich, ergibt die errechnete Gewebsglucosekonzentration eine gute Übereinstimmung mit der gleichzeitig ermittelten Blutglucosekonzentration. Die errechneten Werte der Gewebsglucose liegen etwas niedriger als die gleichzeitig experimentell ermittelten Blutglucosekonzentrationswerte, was mehrere Ursachen hat:

1. In der Perfusionsflüssigkeit findet eine Glycolyse statt, da aus dem Gewebe glycolytische Enzyme in die Perfusionsflüssigkeit gelangen.
2. Die Analyse erfolgte bei diesem Experiment nicht On-Line sondern Off-Line, wobei die Perfusionsflüssigkeit jeweils 15 Minuten akkumuliert und erst dann analysiert wurde. Dadurch ist zum Teil der Unterschied im zeitlichen Verlauf der Blutglucose und der errechneten Gewebsglucose erklärbar. Weitere Experimente haben gezeigt, daß durch Zusatz von konkurrierenden Substraten für ATP eine noch bessere Übereinstimmung der beiden Kurven erzielt werden kann. Die Meßkurven in Fig. 21 stammen von einer gesunden Kontrollperson nach der oralen Gabe von 75 g Glucose.

Fig. 22 zeigt ein anderes Beispiel einer vergleichsweisen Ermittlung der Blutzucker- und Gewebezuckerkurve bei einer Patienten mit Typ 1-Diabetesmellitus. Wie im unteren Teil der Abbildung ersichtlich, liegen auch hier die Natriumionenkonzentrationen in der Perfusionsflüssigkeit zwischen 3 und 30 mmol/l, sodaß auch hier ein ständig wechselnder Equilibrationsgrad demonstriert wird. Die aus der Perfusatglucose und dem Perfusatnatrium errechneten Gewebsglucosewerte zeigen auch hier einen guten, parallelen Verlauf zur gleichzeitig ermittelten Blutglucose. Auffallend ist auch hier eine etwas niedrigere Glucosekonzentration im Gewebe verglichen mit der Blutglucose. Weiters fällt auf, daß zum Zeitpunkt der Insulingabe über die Subcutannadel im Gewebe noch geringere Glucosekonzentrationen verglichen mit den Blutglucosewerten ermittelt wurden, sodaß, neben einer anfänglich besonders starken Glycolyse, eine lokale verstärkte Insulinwirkung mit Aufnahme von Glucose in die Zellen und dementsprechendem Abfall im Extrazellulärraum nicht ausgeschlossen werden kann. Deswegen wird es zumindest vorläufig günstig sein, den Abstand zwischen Austauschkanal und Austrittsstelle für das Insulin möglichst groß zu halten, oder eventuell das Insulin über eine zweite Nadel an anderer Stelle zu verabreichen.

**Patentansprüche**

1. Vorrichtung zur Bestimmung der Konzentration von zumindest einer in lebenden Organismen vorliegenden Substanz, mit einer Einrichtung zur Zu- und Abfuhr einer Perfusionsflüssigkeit, welche eine in den Organismus einbringbare Hohlnadel aufweist, sowie mit einer Auswerteeinheit, **dadurch gekennzeichnet**, daß die als Subcutannadel/-katheter (2) ausgeführte Hohlnadel in das Gewebe des Organismus einbringbar ist und einen in Wandbereichen (9, 9') offenen Austauschkanal (4) aufweist, daß die Einrichtung (5) zur Zufuhr der Perfusionsflüssigkeit und zur Abfuhr nach deren teilweisen Equilibration mit der zu bestimmenden Substanz, vorzugsweise der Glucose, und im Vergleich zur zu bestimmenden Substanz relativ konstanten endogenen oder exogenen Markergrößen mit dem Austauschkanal (4) verbunden ist, sowie daß eine mit der Zu- und Abfuhreinrichtung (5) verbundene Analyseneinrichtung (10) zur Messung der Konzentration der Substanz und einer Markergröße vorhanden ist, welche an die Auswerteeinheit (11) angeschlossen ist, welche bei teilweiser Equilibration die im Gewebe vorliegende Konzentration der zu bestimmenden Substanz ermittelt.

2. Vorrichtung nach Anspruch 1, **dadurch gekennzeichnet**, daß die Einrichtung (5) zur Zu- und Abfuhr der Perfusionsflüssigkeit ein Reservoir (17) für die Perfusionsflüssigkeit, eine zuführende Leitung (3) zum Austauschkanal (4), eine über zumindest eine Öffnung (6) mit dem Austauschkanal (4) verbundene abführende Leitung (7) zur Analyseneinrichtung (10), sowie ggf. einen über eine Leitung (7') an die Analyseneinrichtung (10) angeschlossenen Auffangbehälter (18) für die Perfusionsflüssigkeit aufweist, wobei zumindest eine Druck- und/ oder Saugpumpe (15, 16; 32) zur Umwälzung der Perfusionsflüssigkeit vorhanden ist, sowie daß eine Kalibriereinrichtung (20) zur Kalibrierung der Sensoren in der Analyseneinrichtung (10) vorhanden ist, welche einen Behälter (21) für die Kalibrierlösung und eine durch Ventile (24, 25) steuerbare Verbindungsleitung (22) zur Analyseneinrichtung (10), eine Pumpe (23) für die Kalibrierlösung, sowie ggf. einen Auffangbehälter (43) für die Kalibrierlösung aufweist.

3. Vorrichtung nach Anspruch 1, **dadurch gekennzeichnet**, daß eine Einrichtung (62) zur Zufuhr eines Medikamentes vorhanden ist, welche ein Reservoir (19) für das Medikament, eine Medikamentenpumpe (12) und eine Zuleitung (13) für das Medikament aufweist, wobei eine Medikamentenöffnung (14) in der Zuleitung (13) den Austritt des Medikaments ins Gewebe ermöglicht.

4. Vorrichtung nach Anspruch 1, **dadurch gekennzeichnet**, daß die Subcutannadel/-katheter (2) zumindest zwei Leitungen aufweist, daß eine der Leitungen als mit Öffnungen (9) in Wandbereichen (9, 9') ausgestatteter Austauschkanal (4) und die andere als mit diesen über zumindest eine Öffnung (6) im Bereich der Spitze (1') der Subcutannadel/-katheter (2) verbundene abführende Leitungen (7) für die Perfusionsflüssigkeit ausgebildet ist, wobei ein Großteil der Naderoberfläche als Equilibrationsfläche zur Verfügung steht.

5. Vorrichtung nach Anspruch 4, **dadurch gekennzeichnet**, daß die Subcutannadel/-katheter (2) aus zwei ineinanderliegenden Kanülen (63, 65) mit unterschiedlichen Durchmessern besteht, wobei zwischen den beiden Kanülen (63, 65) ein Austauschkanal (4) mit im wesentlichen ringförmigen Querschnitt entsteht und die innere Kanüle (63) bis nahe an die Spitze (1') der äußeren Kanüle (65) reicht.

6. Vorrichtung nach Anspruch 4, **dadurch gekennzeichnet**, daß der Austauschkanal (4) und die abführende Leitung (7) der Subcutannadel/-katheter (2) durch eine sich in Längsrichtung der Nadel erstreckende Trennwand (30) getrennt sind.

7. Vorrichtung nach Anspruch 4, **dadurch gekennzeichnet**, daß am von der Spitze (1') abgewandten Ende der Subcutannadel/-katheter (2) eine Steckverbindung (26) angeordnet ist, welche den Austauschkanal (4) bzw. die abführende Leitung (7) in der Subcutannadel/-katheter (2) mit der zuführenden Leitung (3) bzw. mit den außerhalb der Nadel liegenden Teil der abführenden Leitung (7) verbindet, wobei aufgrund der geometrischen Konfiguration der Steckverbindung (26) eine eindeutige Zuordnung der in der Subcutannadel/-katheter (2) vorliegenden Leitungen zum übrigen Teil der Vorrichtung möglich ist.

8. Vorrichtung nach Anspruch 4, **dadurch gekennzeichnet**, daß die Subcutannadel/-katheter (2) im Bereich ihrer Spitze (1') eine Medikamentenöffnung (14) aufweist, welche mit der als dritte Leitung in der Subcutannadel/-katheter (2) verlaufenden Zuleitung (13) für das Medikament in Verbindung steht.

9. Vorrichtung nach Anspruch 8, **dadurch gekennzeichnet**, daß die abführende Leitung (7) und die Zuleitung (13) für das Medikament in einer in Längsrichtung durch eine Trennwand (30) geteilten Kanüle (63) verlaufen, welche von einer äußeren Kanüle (65) umgeben ist und mit dieser einen im wesentlichen kreisringförmigen Austauschkanal (4) bildet, wobei die äußere Kanüle (65) über zahlreiche über einen möglichst großen Teil der Oberfläche der Subcutannadel/-katheter (2) verteilte Öffnungen (9) verfügt.

10. Vorrichtung nach Anspruch 8, **dadurch gekennzeichnet**, daß die Zuleitung (13) für das Medikament, die abführende Leitung (7) und der Austauschkanal (4) als im wesentlichen konzentrisch ineinanderliegende Kanülen (63, 64, 65) ausgeführt sind, wobei die innerste Kanüle (63) die Zuleitung (13) für das Medikament ist und im Bereich der Spitze (1') der Subcutannadel/-katheter (2) eine Öffnung (14) zum Gewebe aufweist, daß die äußere Kanüle (65) über zahlreiche über einen möglichst großen Teil der Oberfläche verteilte Öffnungen (9) verfügt, daß die abführende Leitung (7) im Bereich der Spitze (1') der Subcutannadel/-katheter (2) zumindest eine Öffnung (6) zum Austauschkanal (4) aufweist und abführende Leitung (7) sowie Austauschkanal (4) im Bereich der Spitze (1') ggf. blind verschlossen sind.

11. Vorrichtung nach Anspruch 8, **dadurch gekennzeichnet**, daß die Subcutannadel/-katheter (2) eine innere Kanüle (63) zur Aufnahme der abführenden Leitung (7) und ggf. der Zuleitung (13) für ein Medikament aufweist, wobei an der Oberfläche der Kanüle (63) zahlreiche längs oder spiralig verlaufende Septen (27) angeordnet sind, welche nach Einbringen der Kanüle in das Gewebe den Austauschkanal (4) bilden, sowie daß zur Abfuhr der Perfusionsflüssigkeit die abführende Leitung (7) zumindest eine Öffnung (6) zum Raum zwischen den Septen (27) aufweist, sowie daß die Subcutannadel/-katheter (2) aus zum Teil elastischem Material besteht und zum Schutz der Septen (27) in einer Hohlnadel (28) untergebracht ist, welche nach dem Einstechen in das Gewebe entfernbar ist.

12. Vorrichtung nach Anspruch 8, **dadurch gekennzeichnet**, daß die Subcutannadel/-katheter (2) durch ein verzweigtes Septum (31) oder mehrere Septen in Längsrichtung in einen Austauschkanal (4), eine abführende Leitung (7) und eine Zufuhrleitung (13) für das Medikament unterteilt ist, wobei die Subcutannadel/-katheter (2) im Bereich des Austauschkanals (4) über zahlreiche Öffnungen (9) verfügt.

13. Vorrichtung nach Anspruch 4, **dadurch gekennzeichnet**, daß der Austauschkanal (4) der Subcutannadel/-katheter (2) zwischen den Öffnungen (9) brückenförmige Überdachungen (8) zur Abstützung des Gewebes aufweist, sowie daß die vom Austauschkanal (4) zum Gewebe führenden Öffnungen (9) in mehreren, vorzugsweise in vier Reihen, längs der Oberfläche der Subcutannadel/-katheter (2) angeordnet sind und daß der Durchmesser der Öffnungen (9) des Austauschkanals (4) vorzugsweise kleiner ist als der Durchmesser der abführenden Leitung (7).

14. Vorrichtung nach Anspruch 1, **dadurch gekennzeichnet**, daß die Druck- und Saugpumpe (15, 16) der Einrichtung (5) zur Zu- und Abfuhr der Perfusionsflüssigkeit durch eine Kolbenpumpe (32) realisiert ist, wobei der an beiden Seiten abgeschlossene Kolbenraum (37) einen durch den Kolben (33) vom Reservoir (17) getrennten Auffangbehälter (18) für die Perfusionsflüssigkeit bildet, wobei der Antriebsstempel (34) der Kolbenpumpe (32) gegen die Außenwand der Kolbenpumpe abgedichtet ist.

15. Vorrichtung nach Anspruch 14, **dadurch gekennzeichnet**, daß der Antriebsstempel (34) der Kolbenpumpe (32) biegsam ausgeführt ist, durch das Reservoir (17) und den Behälter (18) für die Perfusionsflüssigkeit geführt ist und auf jeder Seite gegen die Wand der Kolbenpumpe (32) abgedichtet ist, sowie daß der Antriebsstempel (34) an Umlenkteilen (66) an der Außenseite der Kolbenpumpe (34) anliegt und in sich geschlossen geführt ist.

**16.** Vorrichtung nach Anspruch 14, **dadurch gekennzeichnet**, daß der Antriebsstempel (34) der Kolbenpumpe (32) drehbar gelagert ist, wobei der Kolben (33) der Drehbewegung des Antriebsstempels aufgrund seiner Formgebung nicht folgt, sowie daß die Drehbewegung des Antriebsstempels durch ein am Antriebsstempel (34) und am Kolben (33) vorhandenes Gewinde (41) in eine Längsbewegung des Kolbens (33) umsetzbar ist.

**17.** Vorrichtung nach Anspruch 14, **dadurch gekennzeichnet**, daß der Antriebsstempel (34) der Kolbenpumpe (32) hohl ist und an der Innenseite ein Gewinde (41') aufweist.

**18.** Vorrichtung nach einem der Ansprüche 1, 4, 8 oder 14, **dadurch gekennzeichnet**, daß ein schreibstift- oder handgriffähnliches Gehäuse (70) vorhanden ist, an welchem die Subcutannadel/-katheter (2) befestigbar ist, daß das Gehäuse (70) die Einrichtung (5) zur Zu- und Abfuhr der Perfusionsflüssigkeit, die damit verbundene Analyseneinrichtung (10) und die Auswerteeinheit (11) aufweist, sowie daß eine an die Auswerteeinheit (11) angeschlossene Anzeige (73) für die Darstellung der gemessenen Parameter am Gehäuse (70) angeordnet ist.

**19.** Vorrichtung nach Anspruch 18, **dadurch gekennzeichnet**, daß im Gehäuse (70) zusätzlich eine Einrichtung (62) zur Zufuhr eines Medikamentes vorhanden ist, wobei die Medikamentenapplikation durch die bereits vorhandene Subcutannadel/-katheter (2) erfolgt.

**20.** Vorrichtung nach einem der Ansprüche 1, 14 oder 18, **dadurch gekennzeichnet**, daß die Kolbenpumpe (32) für die Perfusionsflüssigkeit, die Medikamentenpumpe (12) und ggf. die Pumpe (23) für die Kalibrierflüssigkeit, über eine Hebeleinrichtung (71) manuell betätigbar sind.

**21.** Vorrichtung nach Anspruch 20, **dadurch gekennzeichnet**, daß die Hebeleinrichtung (71) einen Energiespeicher (74), vorzugsweise Federn auflädt, welche in der Folge unter Zwischenschaltung eines Getriebes (72) auf die Pumpen (12, 23; 32) einwirken.

**22.** Vorrichtung zur Bestimmung der Konzentration zumindest einer in lebenden Organismen vorliegenden Substanz mit einer Einrichtung zur Zu- und Abfuhr einer Perfusionsflüssigkeit, sowie einer Auswerteeinheit, **dadurch gekennzeichnet**, daß alle Komponenten der Vorrichtung und deren Energieversorgung in einem implantierbaren Gehäuse angeordnet sind, daß die Oberfläche des Gehäuses zumindest zum Teil als in Wandbereichen (9) offener Austauschkanal (4) ausgebildet ist, daß die Einrichtung (5) zur Zufuhr der Perfusionsflüssigkeit und zur Abfuhr nach deren teilweisen Equilibration mit der zu bestimmenden Substanz, vorzugsweise der Glucose, und im Vergleich zur zu bestimmenden Substanz relativ konstanten endogenen oder exogenen Markergrößen mit dem Austauschkanal (4) verbunden ist, sowie daß eine mit der Zu- und Abfuhreinrichtung (5) verbundene Analyseneinrichtung (10) zur Messung der Konzentration der Substanz und einer Markergröße vorhanden ist, welche an die Auswerteeinheit (11) angeschlossen ist, welche bei teilweiser Equilibration die im Gewebe vorliegende Konzentration der zu bestimmenden Substanz ermittelt, sowie daß ein Reservoir (17) für die Perfusionsflüssigkeit und ein Reservoir (19) für ein Medikament je durch ein Septum (57) transcutan nachfüllbar sind.

**23.** Vorrichtung nach Anspruch 22, **dadurch gekennzeichnet**, daß eine mechanische Einrichtung (59), vorzugsweise Stempel zur Entfernung von in den Austauschkanal (4) einwachsenden Gewebeteilen vorgesehen ist, daß zumindest ein Reservoir (17, 19) über verformbare Wandteile (58) verfügt, wobei die beim Füllen des Reservoirs (17, 19) bewirkte Ausdehnung die Stempel antreibt.

**24.** Vorrichtung nach einem der Ansprüche 1, 3 oder 22, **dadurch gekennzeichnet**, daß die zur Zuschaltung der einzelnen Zu- und Abführleitungen (3, 7, 7', 13, 22) zu den einzelnen Behältern (17, 18, 19, 21, 43) benötigten Ventile (24, 25 25a) durch ein Mehrwegventil (44) realisiert sind, dessen Ventilkörper (44') alle Zu- und Abführleitungen (3, 7, 7', 13, 22) enthält und dessen beweglicher Teil (49) Kanäle (61) bzw. Nuten zu deren eindeutigen Zuordnung aufweist, wobei einzelne Positionen des beweglichen Teils (49) durch ein Stellrad (78) oder automatisch durch an einer Antriebseinheit anliegende Signale der Auswerteeinheit (11) einstellbar sind.

**25.** Vorrichtung nach Anspruch 24, **dadurch gekennzeichnet**, daß der Ventilkörper (44') eine Öffnung (47) aufweist, die mit der Außenluft in Verbindung steht und ein Bakterienfilter (48) aufweist.

**26.** Vorrichtung nach Anspruch 24, **dadurch gekennzeichnet**, daß für den beweglichen Teil (49) des Mehrwegventils (44) zumindest drei Positionen möglich sind, wobei in einer ersten Position gleichzeitig Verbindungen zwischen dem Reservoir (17) für die Perfusionsflüssigkeit und der zuführenden Leitung (3), zwischen der abführenden Leitung (7) und der Analyseneinrichtung (10), zwischen der Analyseneinrichtung (10) und dem Auffangbehälter (18), sowie ggf. zwischen dem Reservoir (19) für das Medikament und der Zuleitung (13) für das Medikament; in einer zweiten Position Verbindungen zwischen der Öffnung (47) zum Ansaugen von Luft und der Analyseneinrichtung (10), sowie zwischen der Analyseneinrichtung (10) und dem Auffangbehälter (18) und in einer dritten Position Verbindungen zwischen dem Behälter (21) für die Kalibrierlösung und der Analyseneinrichtung (10), sowie zwischen der Analyseneinrichtung (10) und dem Auffangbehälter (43) für die Kalibrierlösung herstellbar sind.

27. Vorrichtung nach Anspruch 26, **dadurch gekennzeichnet**, daß in einer vierten Position des beweglichen Teils (49) des Mehrwegventils (44) gleichzeitig Verbindungen zwischen dem Reservoir (17) und der zuführenden Leitung (3), zwischen abführender Leitung (7) und Analyseneinrichtung (10), zwischen Analyseneinrichtung (10) und Auffangbehälter (18), sowie zwischen dem Behälter (21) für die Kalibrierlösung und der Zuleitung (13) für das Medikament herstellbar sind, sowie daß in einer fünften Position des beweglichen Teils (49) des Mehrwegventils (44) zur Nullpunktskalibrierung gleichzeitig Verbindungen zwischen dem Reservoir (17) für die Perfusionsflüssigkeit und der Analyseneinrichtung (10), sowie zwischen der Analyseneinrichtung (10) und dem Auffangbehälter (18) herstellbar sind, daß weiters in einer sechsten Position des beweglichen Teils (49) des Mehrwegventils (44) gleichzeitig Verbindungen zwischen der Öffnung (47) zum Ansaugen von Luft und der zuführenden Leitung (3), sowie zwischen der abführenden Leitung (7) und der Analyseneinrichtung (10), sowie zwischen der Analyseneinrichtung (10) und dem Auffangbehälter (18) herstellbar sind.

28. Vorrichtung nach einem der Ansprüche 2, 18 oder 22, **dadurch gekennzeichnet**, daß in der abführenden Leitung (7) in der zum Auffangbehälter (18) führenden Leitung oder im Auffangbehälter (18) für die Perfusionsflüssigkeit ein Unterdrucksensor (18') vorgesehen ist, sowie daß in der Analyseneinrichtung (10) ein Temperatursensor (10') vorgesehen ist.

29. Vorrichtung nach einem der Ansprüche 1, 18 oder 22, **dadurch gekennzeichnet**, daß der zur Bestimmung der Markergröße eingesetzte Ionensensor oder Leitfähigkeitsmesser (10') in der Analyseneinrichtung (10) als Unterdrucksensor (18') fungiert und die adequate Füllung der Meßkapillare (50) der Analyseneinrichtung (10) registriert, wobei ein Signal der Auswerteeinheit (11) die Um- oder Abschaltung einer Saugpumpe (16) für die Perfusionsflüssigkeit bewirkt und ggf. eine akustische oder optische Anzeige vorgesehen ist.

30. Vorrichtung nach einem der Ansprüche 1, 18 oder 22, **dadurch gekennzeichnet**, daß die Analyseneinrichtung (10) zur Erfassung der zu messenden Substanz und der endogenen oder exogenen Markergrößen als austauschbarer Block vorliegt, bei dessen Ausbau die mit der Analyseneinrichtung (10) in Verbindung stehenden Leitungen (7, 7') über einen Verschließmechanismus (52) abschließbar sind, sodaß der aufgebaute Druck in diesen Leitungen (7,7') erhalten bleibt.

31. Vorrichtung nach einem der Ansprüche 2, 18 oder 22, **dadurch gekennzeichnet**, daß im Behälter (21) der Kalibriervorrichtung (20) eine zum Medikament im Reservoir (19) antagonistisch wirksame Substanz vorliegt, sowie daß eine Leitung zwischen dem Behälter (21) für die Kalibrierlösung und der Zuleitung (13) für das Medikament vorhanden ist.

32. Vorrichtung nach einem der Ansprüche 1, 18 oder 22, **dadurch gekennzeichnet**, daß alle Behälter und Reservoire sowie alle zu- und abführenden Leitungen als Einmal-System auf einem gemeinsamen Träger (45) untergebracht sind, wobei durch das Einsetzen dieses gemeinsamen Trägers (45) in die Vorrichtung der Kraftschluß zwischen den Antrieben (35a, 35b, 35c) von Pumpen und eines Mehrwegventils (44) automatisch herstellbar ist.

33. Vorrichtung nach Anspruch 3 oder 18, **dadurch gekennzeichnet**, daß ein Dosierschalter zur Steuerung der Medikamentendosis vorhanden ist, in dessen ersten Stellung die Dosierung automatisch und kontinuierlich in der von der Auswerteeinheit (11) errechneten Weise erfolgt und in dessen zweiten Stellung eine vom Benutzer vorbestimmte Medikamentendosis applizierbar ist.

**Claims**

1. A device for determining the concentration of one or more substances in living organisms, comprising a unit for feeding and draining a perfusion fluid, which is provided with a hollow needle to be inserted into the organism, and further comprising an evaluation unit, **characterized in that** the hollow needle configured as a hypodermic needle or catheter (2) can be inserted into the tissue of the organism and has an exchange channel (4) with openings (9, 9') along its wall, and that the unit (5) for feeding in the perfusion fluid and draining it after its partial equilibration with the substance to be analyzed, preferably glucose, and with endogenous or exogenous marker variables which are relatively constant compared to the substance to be analyzed, is connected to the said exchange channel (4), and, further, that an analyzing unit (10) connected to the feeder/drainage unit (5) is provided for measuring the concentration of the substance and one marker variable, which analyzing unit (10) is connected to the evaluation unit (11) determining the concentration in the tissue of the substance to be analyzed upon partial equilibration.

2. A device according to claim 1, characterized in that the unit (5) for feeding and draining the perfusion fluid comprises a reservoir (17) for the perfusion fluid, a feeder line (3) towards the exchange channel (4), a drainage line (7) towards the analyzing unit (10), which line (7) is connected with the exchange channel (4) by one or more openings (6), and, if required, a collecting vessel (18) for the perfusion fluid connected to the analyzing unit (10) by means of a line (7'), with at least one pressure and/or suction pump (15, 16; 32) for circulation of the perfusion fluid, and, further, that a calibrating unit (20) is provided for calibration of the sensors in the analyzing unit (10), comprising a container (21) for the calibrating solution and a connecting line (22) into the analyzing unit (10) controlled by valves (24, 25), a pump (23) for the calibrating solution and, if required, a collecting vessel (43) for the calibrating solution.

3. A device according to claim 1, characterized in that a unit (62) for delivering a drug is provided, comprising a drug reservoir (19), a drug delivery pump (12) and a drug feeder line (13), a drug release opening (14) in the feeder line (13) permitting the drug to drain into the tissue.

4. A device according to claim 1, characterized in that the hypodermic needle/catheter (2) has at least two passages or lines, one of which is configured as an exchange channel (4) with openings (9) in wall areas (9, 9'), and the other one as a drainage line (7) for the perfusion fluid, which is connected with the exchange channel by one or more openings (6) near the tip (1') of the hypodermic needle/catheter (2), a large part of the surface of the needle serving as an equilibration area.

5. A device according to claim 4, characterized in that the hypodermic needle/catheter (2) consists of two cannulas (63, 65) of different diameters, one contained within the other, such that an exchange channel (4) of essentially annular cross-section is formed between the two cannulas (63, 65), the inner cannula (63) extending almost as far as to the tip (1') of the outer cannula (65).

6. A device according to claim 4, characterized in that the exchange channel (4) and the drainage line (7) of the hypodermic needle/catheter (2) are separated by a partition wall (30) running along the length of the needle.

7. A device according to claim 4, characterized in that a plug-in connection (26) is provided at the base end of the hypodermic needle/catheter (2), connecting the exchange channel (4), or rather, the drainage line (7) in the hypodermic needle/catheter (2) to the feeder line (3), or rather, to the part of the drainage line (7) located outside the needle, the lines contained in the hypodermic needle/catheter (2) being properly aligned with the lines in the remaining part of the device due to the geometric configuration of the plug-in connection (26).

8. A device according to claim 4, characterized in that the hypodermic needle/catheter (2) has a drug release opening (14) near its tip (1'), the said opening (14) communicating with the drug feeder line (13) contained as a third passage in the hypodermic needle/catheter (2).

9. A device according to claim 8, characterized in that the drainage line (7) and the drug feeder line (13) are contained in a cannula (63), which is divided along its length by a partition wall (30), and which is surrounded by an outer cannula (65), the two cannulas (63, 65) forming an essentially circular exchange channel (4), and the outer cannula (65) being provided with numerous openings (9) distributed over as large an area of the surface of the hypodermic needle/catheter (2) as possible.

10. A device according to claim 8, characterized in that the drug feeder line (13), the drainage line (7) and the exchange channel (4) are configured as essentially concentric cannulas (63, 64, 65), the innermost cannula (63) serving as a feeder line (13) for the drug, with an opening (14) into the tissue in the area of the tip (1') of the hypodermic needle/catheter (2), and that the outermost cannula (65) has numerous openings (9) distributed over as large an area of its surface as possible, and that the drainage line (7) has at least one opening (6) into the exchange channel (4) in the area of the tip (1') of the hypodermic needle/catheter (2), and, further, that the drainage line (7) and the exchange channel (4) are closed off in the area of the said tip (1'), if necessary.

11. A device according to claim 8, characterized in that the hypodermic needle/catheter (2) has an inner cannula (63) comprising the drainage line (7) and, possibly, the feeder line (13) for a drug, the surface of the cannula (63) being provided with a number of septa (27) arranged lengthwise or helically, which will form the exchange channel (4) once the cannula (63) has been inserted into the tissue, and that the drainage line (7) has at least one opening (6) into the space between the septa (27) for drainage of the perfusion fluid, and, further, that the hypodermic needle/catheter (2) is partly made of flexible material and is put into a hollow needle (28) for better protection of the septa (27), which needle (28) may be removed after the tissue has been punctured.

12. A device according to claim 8, characterized in that the hypodermic needle/catheter (2) is divided along its length by a branched septum (31), or several septa, into an exchange channel (4), a drainage line (7) and a feeder line (13) for the drug, the hypodermic needle/catheter (2) having numerous openings (9) in the area of the exchange channel (4).

13. A device according to claim 4, characterized in that the exchange channel (4) of the hypodermic needle/catheter (2) has ridges (8) between the openings (9), which will press against the tissue, and that the openings (9) from the exchange channel (4) into the tissue are arranged in several rows, preferably four, along the surface of the hypodermic needle/catheter (2), and, further, that the diameters of the openings (9) of the exchange channel (4) preferably are smaller than the diameter of the drainage line (7).

14. A device according to claim 1, characterized in that the pressure and suction pump (15, 16) of the unit (5) for feeding and draining the perfusion fluid is configured as a plunger pump (32), the plunger chamber (37), which is closed off at both ends, forming a collecting vessel (18) for the perfusion fluid separated from the reservoir (17) by the plunger (33), and the driving rod (34) of the plunger pump (32) being sealed against the exterior wall of the pump.

15. A device according to claim 14, characterized in that the driving rod (34) of the plunger pump (32) is configured as a flexible element, which is guided through the reservoir (17) and the vessel (18) for the perfusion fluid and is sealed on each side against the wall of the plunger pump (32), and, further, that the driving rod (34) is guided over deflecting elements (66) on the outside of the plunger pump (34) and forms a close loop.

16. A device according to claim 14, characterized in that the driving rod (34) of the plunger pump (32) is configured as a rotatable element, the plunger (33) not taking part in the rotation of the driving rod on account of its shape, and that the rotation of the driving rod (34) is transformed into a longitudinal motion of the plunger (33) by means of threads (41) on the driving rod (34) and the plunger (33).

17. A device according to claim 14, characterized in that the driving rod (34) of the plunger pump (32) is hollow and has a thread (41') on its inside.

18. A device according to any of claims 1, 4, 8 or 14, characterized in that a pen- or handle-shaped housing (70) is provided, to which the hypodermic needle/catheter (2) may be attached, and that the said housing (70) contains the unit (5) for feeding and draining the perfusion fluid, the analyzing unit (10) connected thereto, and the evaluation unit (11), and, further, that a display unit (73) connected to the evaluation unit (11) is provided on the housing (70) for presentation of the measured parameters.

19. A device according to claim 18, characterized in that the housing (70) additionally contains a unit (62) for delivering a drug, the drug being applied by the existing hypodermic needle/catheter (2).

20. A device according to any of claims 1, 14 or 18, characterized in that the plunger pump (32) for the perfusion fluid, the drug delivery pump (12) and, possibly, the pump (23) for the calibrating solution are operated by hand via a lever system (71).

21. A device according to claim 20, characterized in that the lever system (71) charges energy-storage elements (74), preferably springs, which subsequently actuate the pumps (12, 23; 32) via a gear (72).

22. A device for determining the concentration of one or more substances in living organisms, comprising a unit for feeding and draining a perfusion fluid as well as an evaluation unit, characterized in that all components of the device including their energy supply are located in an implantable housing, and that the surface of this housing, or at least part thereof, is configured as an exchange channel (4) with openings (9) in its wall, and that the unit (5) for feeding the perfusion fluid and draining it after its partial equilibration with the substance to be analyzed, preferably glucose, and with endogenous or exogenous marker variables that are relatively constant compared to the substance to be analyzed, is connected to the exchange channel (4), and, further, that an analyzing unit (10) connected to the feeder/drainage unit (5) is provided for measuring the concentration of the substance and one marker variable, which unit (5) is connected to the evaluation unit (11) determining the concentration in the tissue of the substance to be analyzed upon partial equilibration, and that a reservoir (17) for the perfusion fluid and a reservoir (19) for a drug each can be refilled transcutaneously via a septum (57).

23. A device accvording to claim 22, characterized in that mechanical elements (59), preferably stamps, are provided in order to keep the exchange channel (4) free from ingrowing tissue particles, and that at least one reservoir (17, 19) has flexible walls (58), the stamps being actuated by the expansion caused by filling the reservoir (17, 19).

24. A device according to any of claims 1, 3 or 22, characterized in that the valves (24, 25, 25a) required for assigning the individual feeder and drainage lines (3, 7, 7', 13, 22) to the individual reservoirs or containers (17, 18, 19, 21, 43) are combined into a multiway valve (44), whose valve body (44') contains all feeder and drainage lines (3, 7, 7', 13, 22), and whose movable part (49) has channels (1) or grooves for proper connection of the said lines, individual positions of the movable part (49) being adjusted by means of a setting disk (78) or automatically, via signals of the evaluation unit (11) received at a drive unit.

25. A device according to claim 24, characterized in that the valve body (44') has an opening (47), which is in contact with the ambient air and is provided with a bacteriological filter (48).

26. A device according to claim 24, characterized in that the movable part (49) of the multiway valve (44) may be switched to at least three positions, where in the first position simultaneous connections are established between the reservoir 17 for the perfusion fluid and the feeder line (3), between the drainage line (7) and the analyzing unit (10), between the analyzing unit (10) and the collecting vessel (18), and, if necessary, between the drug reservoir (19) and the drug feeder line (13); in the second position connections are established between the air intake opening (47) and the analyzing unit (10), and between the analyzing unit (10) and the collecting vessel (18); in the third position connections are established between the container (21) for the calibrating solution and the analyzing unit (10), and between the analyzing unit (10) and the collecting vessel (43) for the calibrating solution.

27. A device according to claim 26, characterized in that in the fourth position of the movable part (49) of the multiway valve (44) simultaneous connections are established between the reservoir (17) and the feeder line (3), between the drainage line (7) and the analyzing unit (10), between the analyzing unit (10) and the collecting vessel (18), and between the container (21) for the calibrating solution and the drug feeder line (13), and that in the fifth position of the movable part (49) of the multiway valve (44) for zero-point calibration simultaneous connections are established between the reservoir (17) for the perfusion fluid and the analyzing unit (10), and between the analyzing unit (10) and the collecting vessel (18), and, further, that in the sixth position of the movable part (49) of the multiway valve (44) simultaneous connections are established between the air intake opening (47) and the feeder line (3), between the drainage line (7) and the analyzing unit (10), and between the analyzing unit (10) and the collecting vessel (18).

28. A device according to any of claims 2, 18 or 22, characterized in that a negative pressure sensor (18') is provided in the drainage line (7), in the passage leading towards the collecting vessel (18), or in the collecting vessel (18) for the perfusion fluid itself, and that a temperature sensor (10') is provided in the analyzing unit (10).

29. A device according to any of claims 1, 18 or 22, characterized in that the ionic sensor or conductivity meter (10') in the analyzing unit (10) used for determining the marker variable acts as a negative pressure sensor (18'), registering adequate filling of the measuring capillary (50) in the analyzing unit (10), a signal of the evaluation unit (11) effecting the switchover or switch-off of a suction pump (16) for the perfusion fluid, and that an acoustical alarm or optical display may be provided.

30. A device according to any of claims 1, 18 or 22, characterized in that the analyzing unit (10) for measuring the substance to be analyzed and the endogenous or exogenous marker variables is configured as a replaceable unit, during whose removal the lines (7, 7') connected to the analyzing unit (10) may be closed off by means of a shutting mechanism (52), which will help maintain the pressure built up in the said lines (7, 7').

31. A device according to any of claims 2, 18 or 22, characterized in that the container (21) of the calibrating unit (20) contains a substance antagonizing the drug in the reservoir (19), and that there is a connecting line between the container (21) for the calibrating solution and the feeder line (13) for the drug.

32. A device according to any of claims 1, 18 or 22, characterized in that all containers and reservoirs as well as all feeder and drainage lines are configured as a one-way system located on a joint supporting element (45), a flexible connection being automatically established between the drive elements (35a, 35b, 35c) of the pumps and a multiway valve (44) by inserting the joint supporting element (45) into the device.

33. A device according to claim 3 or 18, characterized in that a metering switch is added for regulating the dosage of the drug, which in its first position ensures that the preparation is metered automatically and continuously as calculated by the evaluation unit (11), while a dose of the drug that is predetermined by the user is administered in the second position.

**Revendications**

1. Dispositif pour déterminer la concentration d'au moins une substance présente dans les organismes vivants, avec un appareillage pour envoyer et évacuer un liquide de perfusion, appareillage qui comporte une aiguille creuse insérable dans l'organisme, ainsi qu'avec une unité d'exploitation, caractérisé en ce que l'aiguille creuse réalisée en aiguille sous cutanée/cathéter sous cutané (2) peut être introduite dans le tissu de l'organisme et comporte un canal de diffusion (4) avec des ouvertures (9, 9') eu niveau des parois, en ce que l'appareillage (5) est relié au canal de diffusion (4) pour amener le liquide de perfusion et pour l'évacuer après son équilibrage partiel avec la substance à déterminer, de préférence le glucose, et en comparaison avec la substance à déterminer relativement aux grandeurs de marquage endogènes ou exogènes, ainsi qu'en ce qu'un appareillage d'analyse (10) relié à l'appareillage (5) d'alimentation et d'évacuation existe pour mesurer la concentration de la substance et une grandeur de marquage, appareillage qui est connecté à l'unité d'exploitation (11), laquelle détermine la concentration présente dans le tissu de la substance à déterminer lors de l'équilibrage partiel.

2. Dispositif selon la revendication 1, caractérisé en ce que l'appareillage (5) pour amener et répartir le liquide de perfusion comprend :
   - un réservoir (17) pour le liquide de perfusion,
   - un conduit (3) d'alimentation dirigé vers le canal de diffusion (4),
   - un conduit (7) d'évacuation relié par au moins une ouverture (6) au canal de diffusion (4) et partant de l'appareillage d'analyse (10), ainsi qu'éventuellement,
   - un réservoir collecteur (18) relié à l'appareillage d'analyse (10) par un conduit (7') et destiné au liquide de perfusion, une pompe refoulante et/ou une pompe aspirante (15, 16 ; 32) servant au moins à faire circuler le liquide de perfusion, et en ce qu'un dispositif d'étalonnage (20) est en service pour étalonner les capteurs dans l'appareillage d'analyse (10), dispositif qui relie un réservoir (21) pour la solution d'étalonnage et au conduit de liaison (22) pouvant être commandé par des soupapes (24, 25) et dirigé vers l'appareillage d'analyse (10), et qui comporte une pompe (23) pour la solution d'étalonnage, ainsi qu'éventuellement un réservoir collecteur (43) pour la solution d'étalonnage.

3. Dispositif selon la revendication 1, caractérisé en ce qu'un appareillage (62) existe pour amener un médicament et comprend un réservoir (19) pour le médicament, une pompe pour le médicament (12) et un conduit (13) pour ce dernier, un orifice (14) pour le médicament dans le conduit (13) permettant la pénétration du liquide dans le tissu.

4. Dispositif selon la revendication 1, caractérisé en ce que l'aiguille sous cutanée/cathéter sous cutané (2) comporte au moins deux conduits, en ce qu'un des conduits est réalisé en canal de diffusion (4) muni d'ouvertures (9) dans les zones de paroi (9, 9') et les autres conduits (7) sont réalisés en conduits d'évacuation du liquide de perfusion et sont reliés au premier par au moins un orifice (6) situé dans la zone de la pointe (1') de l'aiguille sous cutanée/cathéter sous cutané (2), une grande partie de la surface de l'aiguille étant ainsi disponible comme surface d'équilibrage.

5. Dispositif selon la revendication 5, caractérisé en ce que l'aiguille sous cutanée/cathéter sous cutané (2) est composée de deux canules (63, 65) situées l'une dans l'autre avec des diamètres différents, un canal de diffusion (4) de section transversale essentiellement annulaire prend naissance entre les doux canules (62, 65) et la canule intérieure (63) arrivant jusqu'au voisinage de la pointe (1') de la canule extérieure (65).

6. Dispositif selon la revendication 4, caractérisé en ce que le canal de diffusion (4) et le conduit d'évacuation (2) de l'aiguille sous cutanée/cathéter sous cutané (2) sont séparés par une paroi (30) s'étendant dans le sens longitudinal de l'aiguille.

7. Dispositif selon la revendication 4, caractérisé en ce qu'à l'extrémité éloignée de la pointe (1') de l'aiguille/cathéter sous cutanés (2) est disposée une liaison d'accouplement (26) qui relie le canal de diffusion (4) respectivement au conduit d'évacuation (7) de l'aiguille/cathéter sous cutanés (2) avec le conduit (3) d'amenée ou respectivement la partie du conduit (7) d'évacuation située hors de l'aiguille, une coordination claire des conduits présents dans l'aiguille/cathéter sous cutanés par rapport à la partie restante des dispositif étant possible en raison de la configuration géométrique de la liaison d'accouplement (26).

8. Dispositif selon la revendication 4, caractérisé en ce que l'aiguille/cathéter sous cutanés (2) comporte dans la zone de sa pointe (1') une ouverture pour médicament (14) qui est en liaison avec le conduit d'amenée de médicament (13) comme troisième conduit se développant dans l'aiguille/cathéter sous cutanés (2).

9. Dispositif selon la revendication 8, caractérisé en ce que le conduit d'évacuation (7) et le conduit d'alimentation de médicament (13) se dévoloppent dans une canule (63) divisée dans le sens longitudinal par une paroi de séparation (30), canule (63) qui est entourée d'une couche extérieure (65) et forme avec celle-ci un canal de diffusion (4) en forme essentiellement d'anneau circulaire, la canule extérieure (65) disposant de nombreux orifices répartis sur une partie aussi grande que possible de la surface aiguille/cathéter sous cutanés (2).

10. Dispositif selon la revendication 8, caractérisé en ce que le conduit d'amenée de médicament (13), le conduit d'évacuation (7) et le canal de diffusion (4) sont réalisés en canules (63, 64, 65) situées l'une dans l'autre de façon essentiellement concentrique, la canule la plus intérieure (63) étant le conduit d'amenée de médicament (13) et comportant dans la zone de pointe (1') de l'aiguille/cathéter sous cutanés (2) un orifice (14) pour le tissu, en ce que la canule extérieure (65) dispose de nombreux orifices sur une partie aussi grande que possible de sa surface, en ce que le conduit (7) d'évacuation comprend dans la zone de la pointe (1') de l'aiguille/cathéter sous cutanés (2) au moins un orifice (6) en direction du canal de diffusion (4) et le conduit d'évacuation (7) ainsi que le canal de diffusion (4) sont fermés dans la zone de la pointe (1') éventuellement de façon aveugle.

11. Dispositif selon la revendication 8, caractérisé en ce que l'aiguille/cathéter sous cutanés (2) comporte une canule intérieure (63) destinée à recevoir le conduit (7) d'évacuation et éventuellement la canalisation d'amenée de médicament (13), canule (63) à la surface de laquelle sont disposés de nombreux "septum" (27) se développant longitudinalement ou en spirale et qui constituent le canal de diffusion (4) après l'entrée des canules dans le tissu, et en ce que pour évacuer le liquide de perfusion, le conduit d'évacuation (7) comporte au moins un orifice (6) en direction de l'espace entre les "septum" (27) ainsi qu'en ce que l'aiguille/cathéter sous cutanés (2) est composée d'un matériau en partie élastique et est insérée dans une aiguille creuse (28) destinée à protéger les "septum" (27), aiguille qui peut être enlevée après l'introduction dans le tissu.

12. Dispositif selon la revendication 8, caractérisé en ce que l'aiguille/cathéter sous cutanés (2) est subdivisée par un septum ramifié (31) ou plusieurs "septum" dans le sens longitudinal en un canal de diffusion (4), un conduit d'évacuation (7) et un conduit d'amenée pour le médicament (13), l'aiguille/cathéter sous cutanés (2) disposant dans la zone du canal de diffusion (4) de nombreux orifices (9).

13. Dispositif selon la revendication 4, caractérisé en ce que le canal de diffusion (4) de l'aiguille/cathéter sous cutanés (2) comporte entre les orifices (9) des recouvrements (8) en forme de pont pour assurer l'appui du tissu, ainsi qu'en ce que les orifices (9) conduisent du canal de diffusion (4) au tissu sont disposés en plusieurs rangées, de préférence en quatre rangées, le long de la surface de l'aiguille/cathéter sous cutanés et en ce que le diamètre des orifices (9) du canal de diffusion (4) est de préférence plus petit que le diamètre du conduit d'évacuation (7).

14. Dispositif selon la revendication 1, caractérisé en ce que la pompe de refoulement et la pompe d'aspiration (15, 16) de l'appareillage (5) sont réalisées pour alimenter et évacuer du liquide de perfusion au moyen d'une pompe à piston (32), la chambre de piston (37) fermée des deux côtés formant un réservoir de réception (18) du liquide de perfusion, séparé du réservoir (17) et la tige de commande (34) de la pompe à piston (32) étant rendue étanche contre la paroi extérieure de la pompe à piston.

15. Dispositif selon la revendication 14, caractérisé en ce que la tige de piston (34) de la pompe (32) réalisée flexible, est guidée à travers le réservoir (17) et le réservoir (18) pour le liquide de perfusion et est rendue étanche sur chaque face vis-à-vis de la paroi de la pompe (32) et en ce que la tige de piston (34) est liée aux pièces de renvoi (66) à l'extérieur de la pompe à piston (32) et est guidée entièrement fermée sur elle-même.

16. Dispositif selon la revendication 14, caractérisé en ce que la tige de commande (34) du piston de pompe (32) est monté avec possibilité de tourner, le piston (33) ne suivant pas le mouvement de rotation de la tige de piston en raison de sa conformation, et en ce que le mouvement de rotation de la tige de commande peut être couverte par un filetage (41) existant sur la tige de piston (34) et sur le piston (34) en un déplacement longitudinal du piston (33).

17. Dispositif selon la revendication 14, caractérisé en ce que la tige de commande (34) de la pompe à piston (32) est creuse et comporte sur sa face intérieure un filetage (41').

18. Dispositif selon l'une des revendications 1, 4, 8 ou 14, caractérisé en ce qu'il existe un boîtier (70) semblable à un crayon ou à une poignée, sur lequel peut être fixée l'aiguille/cathéter sous cutanés (2), en ce que le boîtier (70) comprend l'appareillage (5) pour alimenter et évacuer le liquide de perfusion, l'appareillage d'analyse relié au précédent et l'unité d'exploitation (11), et en ce qu'un affichage (73) joint à l'unité d'exploitation (11) est disposé sur le boîtier pour représenter les paramètres mesurés.

19. Dispositif selon la revendication 18, caractérisé en ce que dans le boîtier (70) il existe en plus un dispositif (62) pour amener un médicament, l'application du médicament se faisant par l'aiguille/cathéter sous cutanés (2) déjà existants

20. Dispositif selon l'une des revendications 1, 14 ou 18, caractérisé en ce que la pompe à piston (32) pour le liquide de perfusion, la pompe à médicament (12) et éventuellement la pompe (23) pour le liquide d'étalonnage, peuvent être commandés manuellement par un mécanisme de levier (71).

21. Dispositif selon la revendication 20, caractérisé en ce que le mécanisme de levier (71) charge un réservoir d'énergie (74), de préférence des ressorts, qui ensuite par insertion d'une transmission (72) agissent sur les pompe (12, 23, 32).

22. Dispositif pour la détermination de la concentration d'au moins une substance existant dans des organismes vivants avec un appareillage pour amener et évacuer un liquide de perfusion, ainsi qu'une unité d'exploitation, caractérisé en ce que tous les composants du dispositif et leur alimentation en énergie sont disposés dans un boîtier pouvant être mis en place, en ce que la surface du boîtier est réalisée au moins en partie en canal de diffusion (4) ouvert dans des zones de paroi (9), en ce que l'appareillage (5) est relié avec le canal de diffusion (4) pour alimenter le liquide de perfusion et pour l'évacuer après son équilibrage partiel avec la substance à déterminer, de préférence le glucose, et en comparaison avec la substance à déterminer relativement aux grandeurs de marquage constantes endogènes ou exogènes, et en ce qu'un appareillage d'analyse (10) relié à l'appareillage (5) d'alimentation et d'évacuation existe pour mesurer la concentration de la substance et une grandeur de marquage, appareillage qui est connecté à l'unité d'exploitation (11), laquelle détermine la concentration de la substance à déterminer présente dans le tissu lors de l'équilibrage partiel, et en ce qu'un réservoir (17) pour le liquide de perfusion et un réservoir (19) pour un médicament peuvent être rechargés chacun à travers un "septum" (57) de façon transcutanée.

23. Dispositif selon la revendication 22, caractérisé en ce qu'un appareillage mécanique (59), de préférence des pistons, est prévu pour éloigner des morceaux de tissu s'enfonçant dans le canal de diffusion (4), en ce qu'au moins un réservoir (17, 19) dispose de parties de parois (58) déformables, dont l'expansion lors du remplissage du réservoir (17, 19) commande les pistons.

24. Dispositif selon l'une des revendications 1, 3 ou 22, caractérisé en ce que les soupapes (24, 25, 25a) nécessaires pour réunir les conduits d'alimentation et d'évacuation aux réservoirs individuels (17, 18, 19, 21, 43) sont réalisés au moyen d'une vanne à plusieurs voies, dont le corps (44') contient tous les conduits d'alimentation et d'évacuation (3, 7, 7', 13, 22) et dont la partie mobile (49) comporte des canaux (61) ou des gorges destinés à les associer d'une manière univoque, les positions individuelles de la pièce mobile (49') étant ajustables par une molette de réglage (78) ou automatiquement par des signaux de l'unité d'exploitation (11) se présentent à une unité de commande.

25. Dispositif selon la revendication 24, caractérisé en ce que le corps de soupape (44') comprend un orifice (47) qui est en communication avec l'air extérieur et un filtre contre les bactéries (48).

26. Dispositif selon la revendication 24, caractérisé en ce que trois positions au moins sont possibles pour la pièce mobile (49) du distributeur à plusieurs voies (44) qui dans une première position peut relier simultanément le réservoir (17) de liquide de perfusion et la conduite (3) d'alimentation, le conduit (7) d'évacuation et l'appareillage d'analyse (10), l'appareillage d'analyse (10) et le réservoir collecteur (18), ainsi qu'éventuellement le réservoir de médicament (19) et le conduit d'amenée du médicament (13) ; dans une deuxième position elle peut établir une liaison entre l'orifice (47) pour l'aspiration d'air et l'appareillage d'analyse (10), ainsi qu'entre l'appareillage d'analyse (10) et le réservoir collecteur (18) et dans une troisième position elle peut mettre en communication le réservoir (21) destiné à la solution d'étalonnage et l'appareillage d'analyse (10), ainsi que l'appareillage d'analyse (10) et le réservoir collecteur (43) pour la solution d'étalonnage.

27. Dispositif selon la revendication 26, caractérisé en ce que dans une quatrième position de la pièce mobile (49) du distributeur à plusieurs voies (44), on peut établir des liaisons simultanément entre le réservoir (17) et le conduit (3) d'alimentation, entre le conduit d'évacuation (7) et l'appareillage d'analyse (10), entre l'appareillage d'analyse (10) et le réservoir collecteur (18), ainsi qu'entre le réservoir (21) de la solution d'étalonnage et la conduite d'amenée (13) du médicament, et en ce que dans une cinquième position de la pièce mobile (49) du distributeur à plusieurs voies (44) pour l'étalonnage du point zéro, on peut simultanément établir des connections entre le réservoir (17) de liquide de perfusion et l'appareillage d'analyse (10), ainsi qu'entre l'appareillage d'analyse (10) et le réservoir collecteur (18), en ce qu'en outre dans une sixième position du distributeur à plusieurs voies (44) on peut établir simultanément des liaisons entre l'orifice (47) pour l'aspiration d'air et le conduit (3), ainsi qu'entre le conduit (7) d'évacuation et l'appareillage d'analyse (10), ainsi qu'entre l'appareillage d'analyse (10) et le réservoir collecteur (18).

28. Dispositif selon l'une des revendications 2, 18 ou 22, caractérisé en ce que dans le conduit (7) d'évacuation, dans le tronçon menant au réservoir collecteur (18) ou dans le réservoir collecteur (18') et en ce que dans l'appareillage d'analyse (10) on a prévu un capteur de température (10').

29. Dispositif selon l'une des revendications 1, 18 ou 22, caractérisé en ce qu'un capteur ionique ou mesureur de conductibilité (10') installé pour la détermination des grandeurs de marquage dans l'appareillage d'analyse (10) fonctionne comme capteur de dépression (18') et enregistre le remplissage adéquat du tube de mesure capillaire (50) de l'appareillage d'analyse (10), un signal de l'unité d'exploitation (11) actionnant le branchement ou le débranchement d'une pompe d'aspiration (16) du liquide de perfusion et éventuellement une indication acoustique ou optique a été prévue.

30. Dispositif selon l'une des revendications 1, 18 ou 22, caractérisé en ce que l'appareillage d'analyse (10) est présent pour détecter la substance à mesurer et les grandeurs de marquage endogènes ou exogènes en tant que bloc interchangeable, lors du démontage duquel les conduits (7, 7') liés à l'appareillage d'analyse (10) peuvent être obturés par un mécanisme de fermeture (52), de sorte que la pression établie reste maintenue dans ces conduits (7, 7').

31. Dispositif selon l'une des revendications 2, 18 ou 22, caractérisé en ce que dans le réservoir (21) du dispositif d'étalonnage (20), est placée une substance active de façon antagoniste par rapport aux médicament du réservoir (19), et en ce que un conduit existe entre le réservoir (21) de la solution d'étalonnage et le conduit d'amenée (13) du médicament.

32. Dispositif selon l'une des revendications 1, 18 ou 22, caractérisé en ce que tous les récipients et réservoirs ainsi que tous les conduits d'amenée et d'évacuation sont logés comme un système unique sur un support commun (45), la liaison mécanique entre les commandes (35a, 35b, 35c) de pompes et d'un distributeur à plusieurs voies pouvant être réalisée en automatique lors de la mise en place de ce support commun dans le dispositif.

33. Dispositif selon la revendication 3 ou 18, caractérisé en ce qu'il existe un interrupteur de dosage pour la commande automatique de la dose de médicament, interrupteur dont la première position assure le dosage de façon automatique et continue de la quantité calculée par l'unité d'exploitation (11) et dont la deuxième position permet d'appliquer une dose de médicament prédéterminée par l'utilisateur.

*Fig. 1*

*Fig. 3*

*Fig. 2*

Fig. 4

Fig. 5

Fig. 6

Fig. 7a

Fig. 7f

Fig. 7b

Fig. 7e

Fig. 7c

Fig. 7d

Fig. 7g

Fig. 8

Fig. 9

Fig. 10

_Fig. 11_

_Fig. 12_

*Fig. 13a*

*Fig. 13b*

*Fig. 13c*

*Fig. 13d*

*Fig. 14*

*Fig. 15*

*Fig. 16*

*Fig. 17*

*Fig. 18*

*Fig. 19*

*Fig. 20*

*Fig. 21*

Fig. 22

37